# EUROPEAN PATENT SPECIFICATION

(11) **EP 1 390 075 B1**
(45) Date of publication and mention of the grant of the patent: **18.01.2012**
(21) Application number: 02731264.4
(22) Date of filing: 05.04.2002
(51) Int. Cl.: C12N 15/67, C12N 15/861, A61K 48/00, C12N 15/00, C12N 15/09, C12N 15/63, C12N 15/85, C07H 21/00, A61P 35/04

(54) **CHEMOTHERAPEUTIC INDUCTION OF EGR-1 PROMOTER ACTIVITY IN GENE THERAPY**
CHEMOTHERAPEUTISCHE EINLEITUNG DER EGR-1-PROMOTER-AKTIVITÄT IN GENTHERAPIE
INDUCTION CHIMIOTHERAPEUTIQUE DE L'ACTIVITE DU PROMOTEUR EGR-1 DANS LA THERAPIE GENIQUE

(30) Priority: 06.04.2001 US 282040 P
(43) Date of publication of application: 25.02.2004
(73) Proprietor: The University of Chicago, Chicago, IL 60637 (US); DANA-FARBER CANCER INSTITUTE, Boston, MA 02115 (US)
(72) Inventor: WEICHSELBAUM, Ralph, R., Chicago, IL 60614 (US); KUFE, Donald, W., Wellesley, MA 02181 (US); GUPTA, Vinay, Chicago, IL 60637 (US); MAUCERI, Helen, Wheaton. IL 60187 (US); PARK, James, Chicago, IL 60616 (US); POSNER, Mitchell, Chicago, IL 60657 (US)
(74) Representative: Kremer, Simon Mark
(86) International application number: PCT/US2002/010733
(87) International publication number: WO 2002/080849

(56) References cited:
- PARK JAMES O ET AL: "Transcriptional control of viral gene therapy by cisplatin" JOURNAL OF CLINICAL INVESTIGATION, vol. 110, no. 3, August 2002 (2002-08), pages 403-410, XP002446418 ISSN: 0021-9738
- DATTA RAKESH ET AL: "Reactive oxygen intermediates target CC(A/T)-6GG sequences to mediate activation of the early growth response 1 transcription factor gene by ionizing radiation" PROCEEDINGS OF THE NATIONAL ACADEMY OF SCIENCES OF THE UNITED STATES OF AMERICA, vol. 90, no. 6, 1993, pages 2419-2422, XP002446419 ISSN: 0027-8424
- OHBA MOTOI ET AL: "Production of hydrogen peroxide by transforming growth factor-beta-1 and its involvement in induction of egr-1 in mouse osteoblastic cells" JOURNAL OF CELL BIOLOGY, vol. 126, no. 4, 1994, pages 1079-1088, XP002446420 ISSN: 0021-9525
- JOKI TATSUHIRO ET AL: "Modification of Doxorubicin molecule enhanced the early growth response gene 1 promoter activity" JIKEIKAI MEDICAL JOURNAL, vol. 43, no. 1, 1996, pages 1-7, XP009088101 ISSN: 0021-6968
- ARAI MASASHI ET AL: "Mechanism of doxorubicin-induced inhibition of sarcoplasmic reticulum Ca2+-ATPase gene transcription" CIRCULATION RESEARCH, vol. 86, no. 1, 7 January 2000 (2000-01-07), pages 8-14, XP002446421 ISSN: 0009-7330
- DOROSHOW J H: "PREVENTION OF DOXORUBICIN-INDUCED KILLING OF MCF-7 HUMAN BREAST CANCER CELLS BY OXYGEN RADICAL SCAVENGERS AND IRON CHELATING AGENTS" BIOCHEMICAL AND BIOPHYSICAL RESEARCH COMMUNICATIONS, vol. 135, no. 1, 1986, pages 330-335, XP009088114 ISSN: 0006-291X
- SODHI A ET AL: "INCREASED RELEASE OF HYDROGEN PEROXIDE AND SUPEROXIDE ANION BY MURINE MACROPHAGES IN-VITRO AFTER CISPLATIN TREATMENT" INTERNATIONAL JOURNAL OF IMMUNOPHARMACOLOGY, vol. 8, no. 7, 1986, pages 709-714, XP002446422 ISSN: 0192-0561
- SULKOWSKA MARIOLA ET AL: "Cyclophosphamide-induced generation of reactive oxygen species. Comparison with morphological changes in type II alveolar epithelial cells and lung capillaries" EXPERIMENTAL AND TOXICOLOGIC PATHOLOGY, vol. 50, no. 3, June 1998 (1998-06), pages 209-220, XP009088115 ISSN: 0940-2993
- UETA EISAKU ET AL: "Manganese superoxide dismutase negatively regulates the induction of apoptosis by 5-fluorouracil, peplomycin and gamma-Rays in squamous cell carcinoma cells" JAPANESE JOURNAL OF CANCER RESEARCH, vol. 90, no. 5, May 1999 (1999-05), pages 555-564, XP009088120 ISSN: 0910-5050
- VAN DER DONK WILFRED A ET AL: "Detection of a new substrate-derived radical during inactivation of ribonucleotide reductase from Escherichia coli by gemcitabine 5'-diphosphate" BIOCHEMISTRY, vol. 37, no. 18, 5 May 1998 (1998-05-05), pages 6419-6426, XP002446423 ISSN: 0006-2960
- BONAVIDA B ET AL: "SYNERGY IS DOCUMENTED IN-VITRO WITH LOW-DOSE RECOMBINANT TUMOR NECROSIS FACTOR CISPLATIN AND DOXORUBICIN IN OVARIAN CANCER CELLS" GYNECOLOGIC ONCOLOGY, vol. 38, no. 3, 1990, pages 333-339, XP002446424 ISSN: 0090-8258
- SEUNG ET AL.: 'Genetic radiotherapy overcomes tumor resistance to cytotoxic agents' CANCER RESEARCH vol. 55, 01 December 1995, pages 5561 - 5565, XP002955986
- WEICHSELBAUM ET AL.: 'Gene therapy targeted by radiation preferentially radiosensitizes tumor cells' CANCER RESEARCH vol. 54, 15 August 1994, pages 4266 - 4269, XP002932030
- STABA ET AL.: 'Adenoviral TNF-alpha gene therapy and radiation damage tumor vasculature in a human malignant glioma xenograft' GENE THERAPY vol. 5, March 1998, pages 293 - 300, XP002955980
- MAUCERI ET AL.: 'Tumor necrosis factor alpha (TNF-alpha) gene therapy targeted by ionizing radiation selectively damages tumor vasculature' CANCER RESEARCH vol. 54, 01 October 1996, pages 4311 - 4314, XP002936211
- CARUSO M.: 'Gene therapy against cancer and HIV infection using the gene encoding herpes simplex virus thymidine kinase' MOLECULAR MEDICINE TODAY vol. 1, May 1996, pages 212 - 217, XP000670302
- HARBOUR ET AL.: 'Rb function in cell-cycle regulation and apoptosis' NATURE CELL BIOLOGY vol. 2, April 2000, pages E65 - E67, XP002955774
- KREITMAN ET AL.: 'Targeting pseudomonas exotoxin to hematologic malignancies' CANCER BIOLOGY vol. 6, 1995, pages 297 - 306, XP002955775
- PEREZ R.P.: 'Cellular and molecular determinants of cisplatin resistance' EUROPEAN JOURNAL OF CANCER vol. 34, no. 10, 1998, pages 1535 - 1542, XP004285043
- SARTORIUS ET AL.: 'Molecular mechanisms of death-receptor-mediated apoptosis' CHEMBIOCHEM. vol. 2, 2001, pages 20 - 29, XP002955776
- GONZALEZ ET AL.: 'Is cisplatin-induced cell death always produced by apoptosis?' MOLECULAR PHARMOCOLOGY vol. 59, 2001, pages 657 - 663, XP002955777
- MYERS C: "The role of iron in doxorubicin-induced cardiomyopathy.", SEMINARS IN ONCOLOGY AUG 1998 LNKD- PUBMED:9768818, vol. 25, no. 4 Suppl 10, August 1998 (1998-08), pages 10-14, ISSN: 0093-7754

## Description

### BACKGROUND OF THE INTENTION

The present application claims priority to co-pending U.S. Patent Application Serial No. 60/282,040, filed April 6, 2001.

### 1. Field of the Invention

The present invention relates generally to the fields of molecular biology and cancer therapy. More particularly, it concerns use of the DNA damaging chemicals to induce expression of the Egr-1 promoter. This permits tissue specific expression of therapeutic genes which, in combination with the DNA damaging chemicals, provide therapy to patients suffering from cancer.

### 2. Description of Related Art

Certain cancer treatment methods, including radiotherapy and chemotherapy, involve damaging the DNA of the cancer cell. The cellular response to normal DNA damage includes activation of DNA repair, cell cycle arrest and lethality (Hall, 1988). For example, the induction of DNA double-strand breaks results in lethal chromosomal aberrations that include deletions, dicentrics, rings, and anaphase bridges (Hall, 1994).

Another approach to treating cancers is gene therapy. This involves the transfer of a foreign gene into a cancer cell, often a tumor suppressor or inducer of apoptosis, under conditions suitable for expression of the gene. Once expressed, the gene product confers a beneficial effect on the tumor cell by either slowing its growth, inhibiting its metastatic potential, or killing it outright.

Combining one or more of these methods is a powerful tool as heterogeneity in many tumors makes mono-therapies far less effective than combinations. However, radio-, chemo- and gene therapy all have the potential for toxic effects. Thus, being able to reduce toxicity, for example, by reducing the amount of radiation/drug/vector administered, is highly advantageous.

For example, tumor necrosis factor-alpha (TNF-α), which has antitumor properties, has been studied as a systemic gene-therapy treatment for cancer in phase 1 studies, but toxicity has limited the therapeutic index of this cytokine (Spriggs *et al*., 1988; Demetri *et al*.,1989) Also, combinations of systemic TNF-α and chemotherapy have been investigated in a few clinical trials with limited success (Nakamoto *et al*., 2000).

On the other hand, chemotherapeutic agents such as cisplatin and other platinum analogues are currently employed in the treatment of several cancers including head and neck, esophageal, lung, testis, ovarian, and bladder cancers. Additionally, cisplatin is used concurrently with irradiation (IR) as a radiosensitizer. In spite of the relative efficacy off cisplatin, tumor-resistance has limited the role of cisplatin in curative cancer chemotherapy (Johnson and Stevenson, 2001). Tumor-derived mechanisms of cisplatin-resistance include an increase in DNA repair of cisplatin adducts in tumor cells, an increase in glutathione, which inhibits free-radical formation and subsequent DNA damage, and a relative decrease in uptake of cisplatin by resistant cells (Kartalou and Essigmann, 2001). The combination of cisplatin with other chemotherapeutic agents, especially 5-FU and VP-16, has increased the therapeutic index of both agents in some human tumors (Kucuk *et al*., 2000), but other strategies are needed to increase the efficacy of cisplatin.

Thus, there is a need in the art to improve both gene-therapeutic as well as chemotherapeutic treatment regimens. Therapies that combine the benefits of different treatment regimens, at the same time reducing the associated side-effects, are desired.

### SUMMARY OF THE INVENTION

The present invention overcomes the deficiencies in the art and provides uses that enhance the therapeutic utility of gene-therapy as well chemotherapy. A transcriptional targeting strategy has been developed wherein inducible expression vectors that encode for therapeutic genes are induced by chemotherapeutic agents. The chemotherapeutic agents specifically target inducible promoters of the expression vector to provide targeted therapy. The therapeutic uses provided are especially effective in treating tumors.

Therefore, in accordance with the present invention, there is provided an expression vector in combination with at least are free-radical inducing DNA damaging compound for use in therapy according to claim 1.

The cell may be a cancer cell, for example, a lung cancer cell, prostate cancer cell, ovarian cancer cell, testicular cancer cell, brain cancer cell, skin cancer cell, colon cancer cell, gastric cancer cell, esophageal cancer cell, tracheal cancer cell, head & neck cancer cell, pancreatic cancer cell, liver cancer cell, breast cancer cell, ovarian cancer cell, lymphoid cancer cell, leukemia cell, cervical cancer cell, or vulvar cancer cell.

The expression vector may further comprise an origin of replication, a selectable marker, or a polyadenylation signal operable linked to the nucleic segment. The expression vector may be plasmid or a viral vector, for example, an adenoviral vector, an adeno-associated viral vector, a retroviral vector, a lentiviral vector, a vaccinia viral vector, or a herpesviral vector. The viral vector may lack one or more viral genes, thus rendering the viral vector non-replicative. The cell may be located in an organism, for example, a human.

The protein of interest may be a tumor suppressor, an inducer of apoptosis, an enzyme, a toxin, a cytokine, or any other protein with antitumor activity. Examples of tumor suppressors are Rb, p16, p53, PTEN, MDA7 or BRCA1 or BRCA2. Examples of inducers of apoptosis are Bax, Bad, Bik, AdE1B, Bim, Bcl-X₈, Bak, TRAIL, Harakiri or Bid. Examples of enzymes are thymidine kinase, cytosine deaminase, hypoxanthine guanine phosphoribosyl transferase. Examples of toxin are pseudomonas exotoxin, diptheria toxin, cholera toxin, pertussis toxin A subunit, enterotoxin A, or ricin A chain. Other molecules with antitumor activity include interleukins (IL) and cytokines exemplified by, IL-1, IL-2, IL-3, IL-4, IL-5, IL-6, IL-7, IL-8, IL-9, IL-10, IL-11, IL-12, IL-13, IL-14, IL-15, β-interferon, α-interferon, γ-interferon, angiostatin, thrombospondin, endostatin, METH-1, METH-2, GM-CSF, G-CSF, M-CSF and tumor necrosis factos (TNF) such as TNF-α and TNF-β. The skilled artisan will recognize that the invention is not limited by any particular protein of interest, such as those disclosed above, as long as the protein has an antitumor effect.

Described herein are methods for treating cancer in a subject comprising (a) providing an expression construct comprising a nucleic acid segment encoding a cancer therapeutic protein, the nucleic acid segment being positioned under the control of an Egr-1 promoter; and (b) administering the expression construct to the subject in combination with a free radical-inducing DNA damaging compound, whereby the DNA damaging compound induces expression of the cancer therapeutic protein from the Egr-1 promoter, thereby treating the cancer in the subject. The expression construct may be delivered local or regional to a tumor located in the subject, delivered systemically, or delivered via intratumoral injection or by direct injection into tumor vasculature.

The DNA damaging compound may be administered prior to administering the expression vector, after administering the expression vector, or at the same time as the expression vector. The expression vector and or DNA damaging agent may be administered at least twice. The cancer therapeutic protein may be a tumor suppressor, an inducer of apoptosis, an enzyme, a toxin, a cytokine, or any protein with anti-tumor activity.

Described herein are methods for inhibiting tumor cell growth in a subject comprising (a) providing an expression construct comprising a nucleic acid segment encoding a cancer therapeutic protein, the nucleic acid segment being positioned under the control of an Egr-1 promoter; and (b) administering the expression construct to the subject in combination with a free radical-inducing DNA damaging compound, whereby the DNA damaging compound induces expression of the cancer therapeutic protein from the Bgr-1 promoter, thereby inhibiting tumor cell growth in the subject, In one such embodiment, the cancer therapeutic protein is TNF-α. In another such embodiment, the free radical-inducing DNA damaging compound is cisplatin.

Described herein are methods for killing a tumor cell in a subject comprising (a) providing an expression construct comprising a nucleic acid segment encoding a cancer therapeutic protein, the nucleic acid segment being positioned under the control of an Egr-1 promoter; and (b) administering the expression construct to the subject in combination with a free radical-inducing DNA damaging compound, whereby the DNA damaging compound induces expression of the cancer therapeutic protein from the Egr-1 promoter, thereby killing the tumor cell in the subject.

Described herein are methods for inhibiting tumor cell metastasis in a subject comprising (a) providing an expression construct comprising a nucleic acid segment encoding a cancer therapeutic protein, the nucleic acid segment being positioned under the control of an Egr-1 promoter; and (b) administering the expression construct to the subject in combinations with a free radical-inducing DNA damaging compound, whereby the DNA damaging compound induces expression of the cancer therapeutic protein from the Egr-1 promoter, thereby inhibiting tumor cell metastasis in the subject.

Described herein are methods for reducing tumor burden in a subject comprising (a) providing an expression construct comprising a nucleic acid, segment encoding a cancer therapeutic protein, the nucleic acid segment being positioned under the control of an Egr-1 promoter; and (b) administering the expression construct to the subject in combination with a free radical-inducing DNA damaging compound, whereby the DNA, damaging compound induces expression of the cancer therapeutic protein from the Egr-1 promoter, thereby reducing tumor burden in the subject.

Described herein are methods for rendering an inoperable tumor operable comprising (a) providing an expression construct comprising a nucleic acid segment encoding a cancer therapeutic protein, the nucleic acid segment being positioned under the control of an Egr-1 promoter; and (b) administering the expression construct to the subject in combination with a free radical-inducing DNA damaging compound, whereby the DNA damaging compound induces expression of the cancer therapeutic protein from the Egr-1 promoter, thereby reducing the size or shape of the tumor and rendering susceptible to resection.

As used herein the specification, "a" or "an" may mean one or more. As used herein in the claim(s), when used in conjunction with the word "comprising", the words "a" or "an" may mean one or more than one. As used herein "another" may mean at least a second or more.

Other objects, features and advantages of the present invention will become apparent from the following detailed description.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

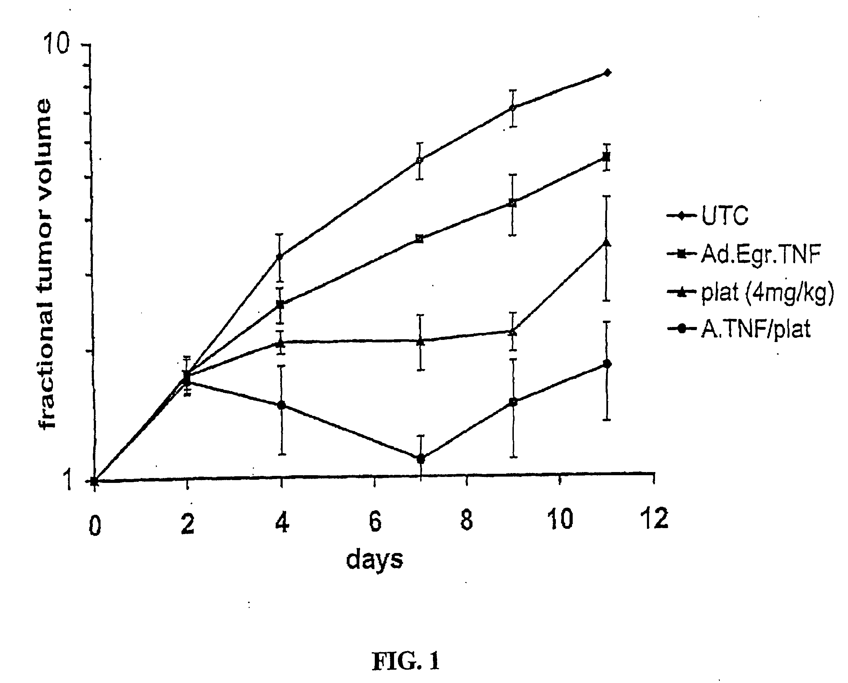

**FIG. 1****. Chemoinduction in Seg-1.** Fractional tumor volume is measured as a function of time and treatment (Seg-1 = esophageal carcinoma cell line; UTC = untreated control; Ad.Egr.TNF = adenovirus encoded tumor necrosis factor under control of the Egr-1 promoter; plat = cisplatinum at 4 mg/kg; A.TNF/plat = Ad.Egr.TNF + plat).

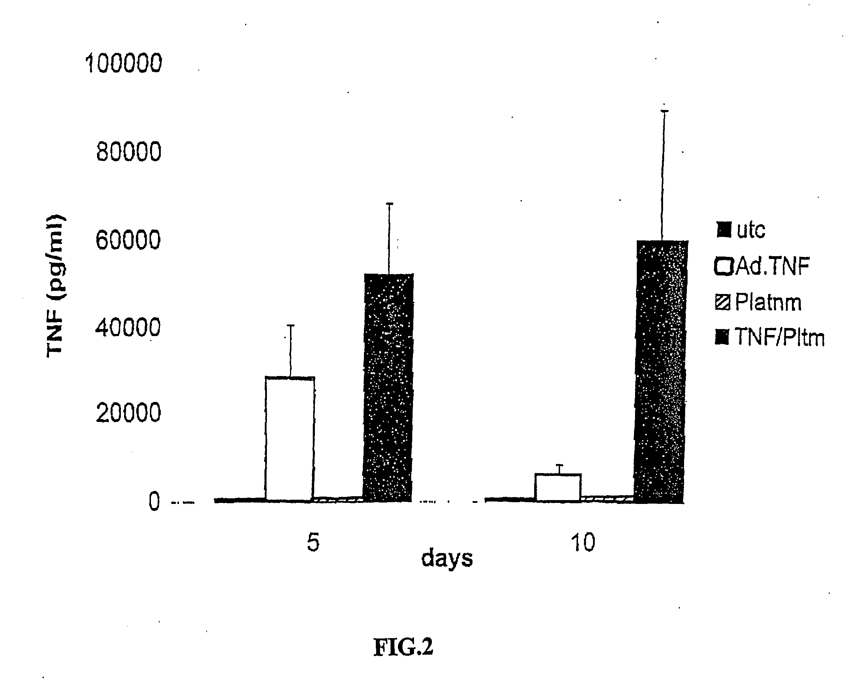

**FIG. 2****. TNF expression with chemoinduction.** TNF production in picograms per ml as a function of time (5 days or 10 days) and treatment (utc = untreated control; Ad.TNF = adenovirus encoded tumor necrosis factor under control of the Egr-1 promoter; Platnm = cisplatinum; TNF/Pltm = Ad.TNF + Platnm).

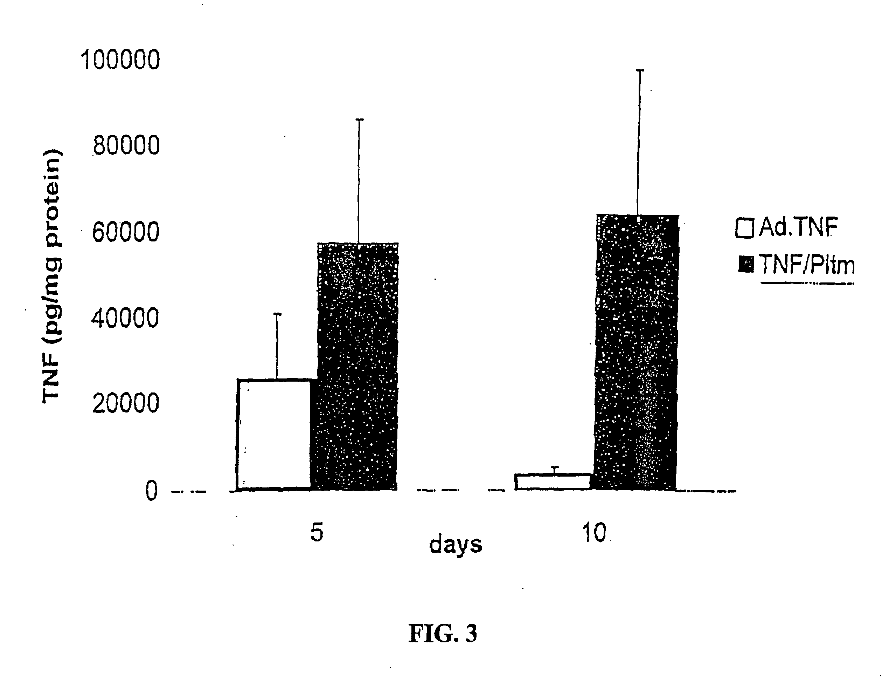

**FIG. 3****. TNF induction with 4 mg/kg platinum.** TNF production in picograms per mg protein as a function of time (5 days or 10 days) and treatment (Ad.TNF = adenovirus encoded tumor necrosis factor under control of the Egr-1 promoter; TNF/Pltm = Ad.TNF + cisplatinum).

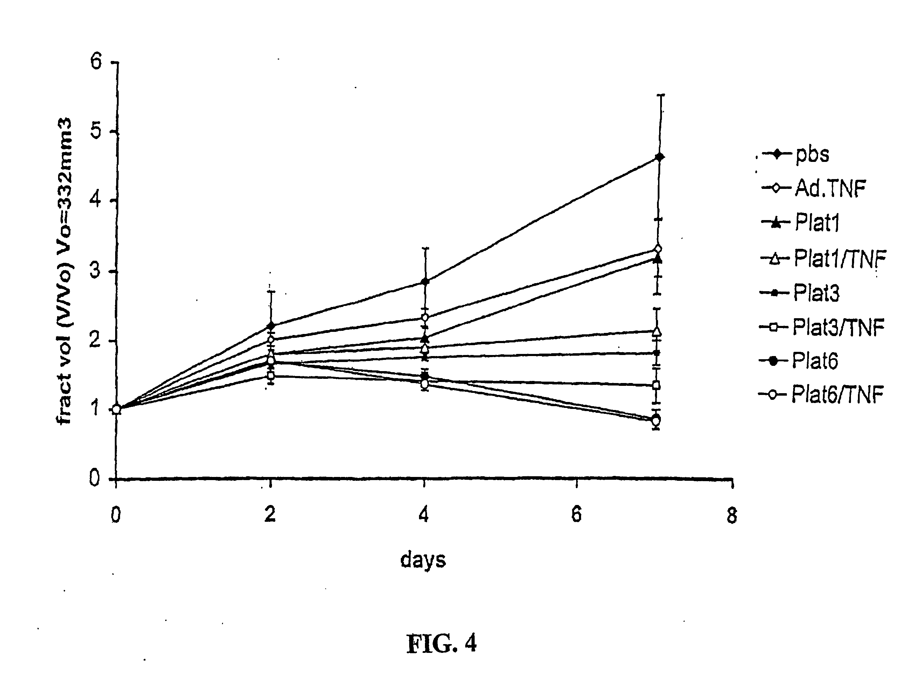

**FIG. 4****. Dose response of platinum in Ad.Egr.TNF-treated Seg-1.** Fractional tumor volume is measured as a function of time and treatment (Seg-1 = esophageal carcinoma cell line; pbs = phosphate buffered saline; Ad.TNF = adenovirus encoded tumor necrosis factor under control of the Egr-1 promoter, Plat1 = cisplatinum at 1 mg/kg; Plat3 = cisplatinum at 3 mg/kg; Plat6 = cisplatinum at 6 mg/kg; Plat1/TNF = cisplatinum at 1 mg/kg + Ad.TNF; Plat3/TNF = cisplatinum at 3 mg/kg + Ad.TNF; Plat6/TNF = cisplatinum at 6 mg/kg + Ad.TNF).

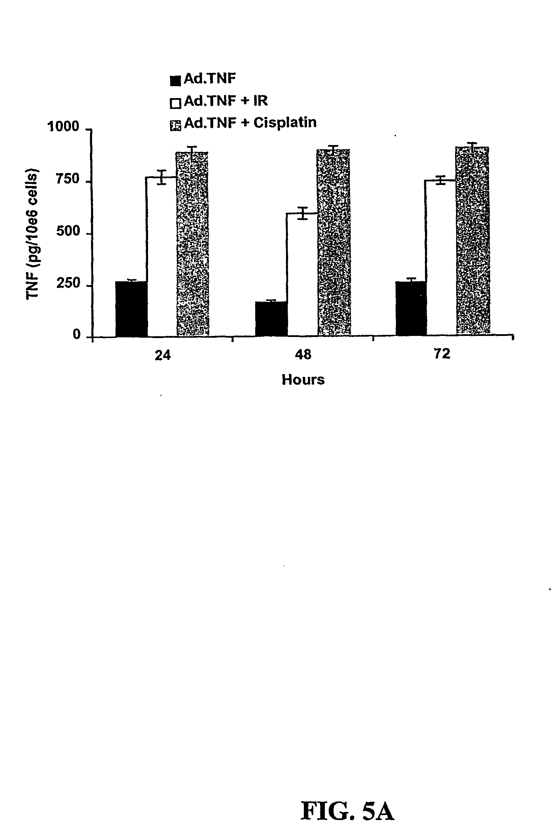

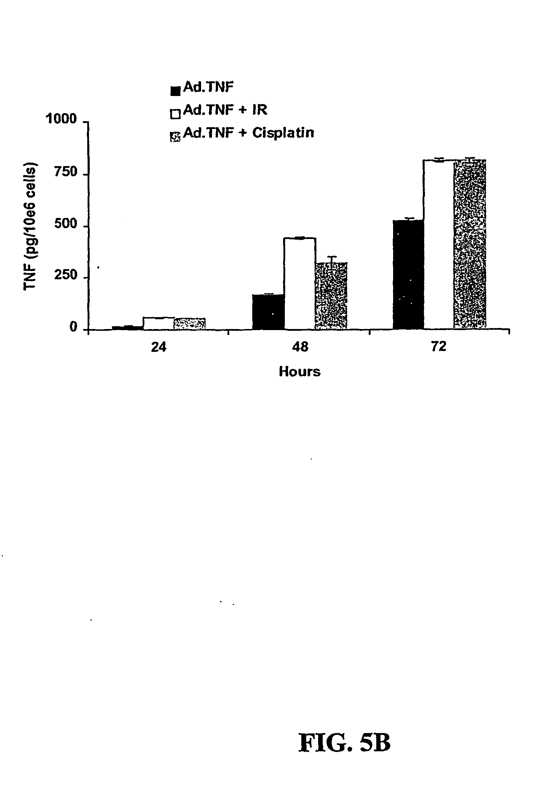

**FIGS. 5A** **&** **5B****. *In vitro* measurement of TNF-α protein.** TNF-α production by Ad.Egr.TNF.11D-infected cells exposed to IR (5 Gy) or cisplatin (5 µM) was measured using ELISA. Significant levels of TNF-α protein were detected at 24, 48 and 72 hrs following exposure to Ad.Egr.TNF.11D + IR (*P*< 0.001) and Ad.Egr.TNF.11D + cisplatin (*P*< 0.001) compared with vector alone in Seg-1 cultures (FIG. 5A) and PROb cultures (FIG. 5B). Data are reported as mean ± SEM.

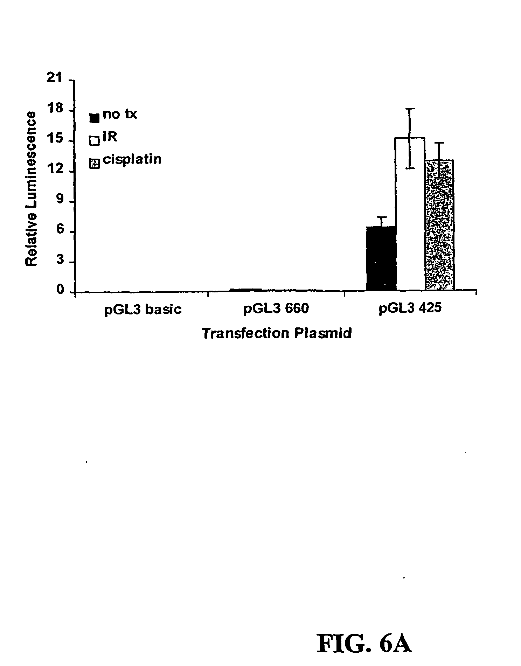

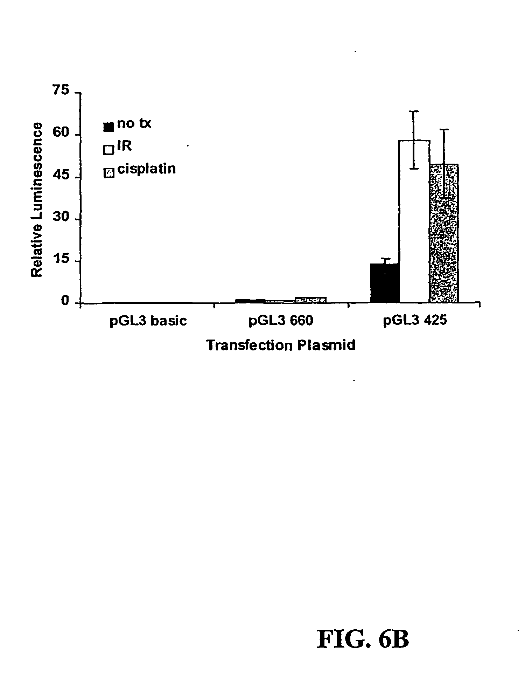

**FIGS. 6A** **&** **6B****. *In vitro* reporter assays.** Luciferase reporter constructs were used to evaluate induction of the Egr-1 promoter by IR or cisplatin. Minimal luciferase activity was detectable following transfection with either the pGL3 (negative control) or the pGL3 660 plasmid (minimal Egr-1 promoter) constructs. FIG. 6A. In Seg-1 cells, a 2.4-fold increase (*P*=0.005) in relative luciferase activity was observed following exposure to IR (20 Gy) and a 2.0-fold increase (*P*=0.005) following exposure to cisplatin (50 µM). FIG. 6B. In PROb cells, a 4.2-fold increase (*P*=0.004) in relative luciferase activity was observed following exposure to IR (20 Gy) and a 3.6-fold increase (*P*=0.01) following exposure to cisplatin (50 µM). Data are reported as mean ± SEM.

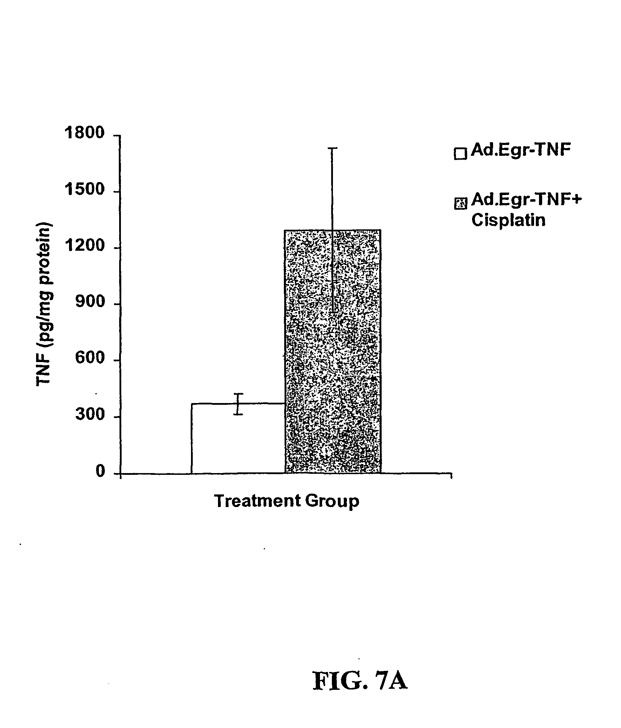

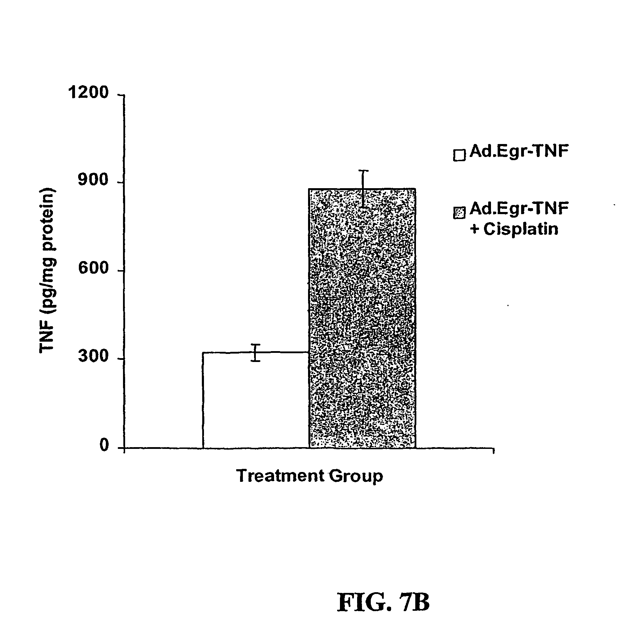

**FIGS. 7A** **&** **7B****. *In vivo* measurement of TNF-α protein.** TNF-α production by Ad.Egr.TNF.11D-injected xenografts was measured by ELISA. A significant increase in intratumoral TNF-α protein concentration was observed following combined treatment with Ad.Egr.TNF.11D + cisplatin compared with treatment with Ad.Egr.TNF.11D vector alone in Seg-1 (FIG. 7A) (3.5-fold increase; *P*<0.05) and PROb (FIG. 7B) xenografts (2.7-fold; *P*<0.001). Data are reported as mean ± SEM.

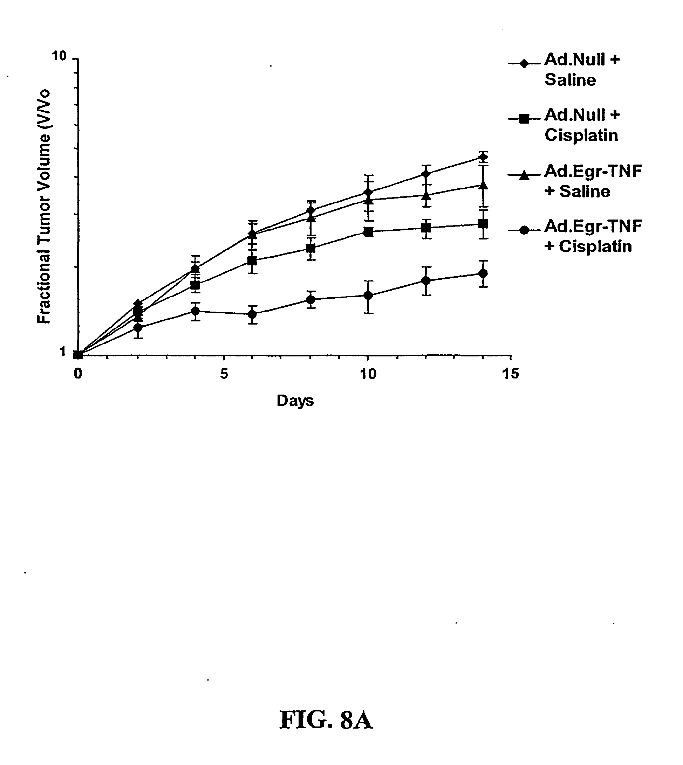

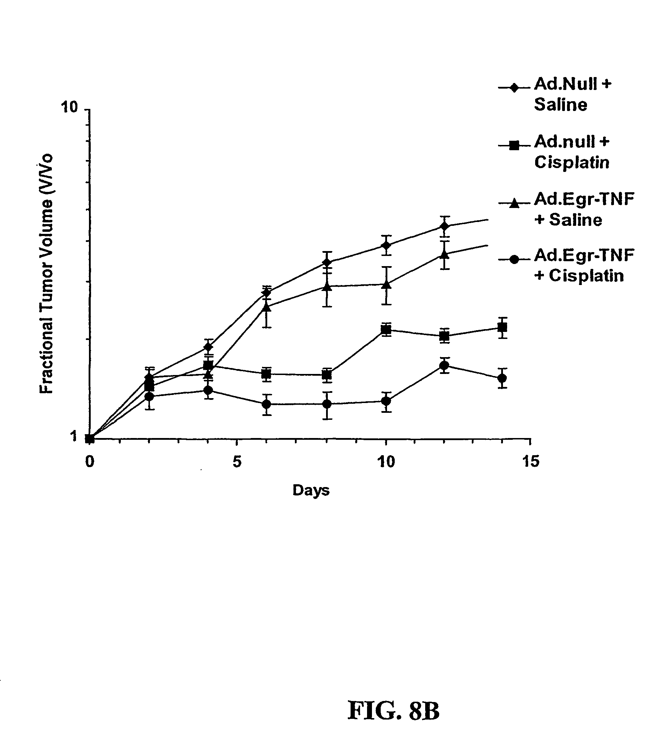

**FIGS. 8A** **&** **8B****. *In vivo* regrowth studies.** The effect of combined treatment with Ad.Egr.TNF.11D and cisplatin was evaluated by measuring the volume of xenograffis injected with Ad.Null.3511.11D or Ad.Egr.TNF.11D with or without cisplatin. FIG. 8A. In Seg-1 xenografts combined treatment with Ad.Egr.TNF.11D + cisplatin produced significant tumor regression compared with tumors treated with the Ad.Null + cisplatin at days on days 4 (*P*=0.045), 6 (*P*<0.005), 8 (*P*<0.002), 10 (*P*<0.001), 12 (*P*<0.004), and 14 (*P*<0.021). FIG. 8B. In PROb xenografts significant tumor regression was observed in the tumors receiving combined treatment with Ad.Egr.TNF.11D + cisplatin compared with tumors treated Ad.Null + cisplatin at days on days 4 (*P*=0.045), 6 (*P*<0.001), 8 (*P*=0.048), 10 (*P*<0.001), 12 (*P*<0.001), and 14 (*P*=0.002). Data are reported as mean ± SEM.

### DESCRIPTION OF ILLUSTRATIVE EMBODIMENTS

The present invention stems in part from the inventors' observation that the Egr-1 promoter, long known to contain radiation-responsive elements, also may be induced by DNA damaging chemicals. This surprising observation provides for a previously unattempted combination therapy for hyperproliferative diseases such as cancer - using an expression construct containing the Egr-1 promoter encoding an antitumor gene such a tumor necrosis factor (TNF) in conjunction with a DNA damaging chemical.

The combined therapeutic effect of the DNA damaging agent as claimed and induced expression of the therapeutic gene in cancer cells provides a superior result to use of either agent alone and also allows for using reduced doses of each agent. Being able to reduce any systemic toxicity, by reducing the amount of radiation and/or drug and/or vector administered, is highly advantageous. The following disclosure provides a detailed description of the foregoing embodiments, as well as variations thereof.

A transcriptional targeting strategy is provided whereby chemotherapeutic agents in conjunction with inducible expression vectors that encode for genes with antitumor effects may be used to effectively treat tumors, where the vectors are induced by the chemotherapeutic agent. Thus, expression constructs comprising the inducible Egr-1 promoter and encoding for any antitumor gene in conjunction with a chemotherapeutic agent that can induce and activate the Egr-1 promoter, via DNA damage or production of ROI's, are provided.

With a selective tumor-targeting vector, a genetic construct that expresses an antitumor gene that is inducible by a chemotherapeutic enhances the effects of the chemotherapeutic as well as the antitumor agent. As both the chemotherapeutic agent and the antitumor gene will generally have different mechanisms of tumor cell killing therefore, cells resistant to one agent may be sensitive to the other. It is also contemplated that such combinations may enhance the local effects of combination chemo-radiation therapy or other adjunct cancer therapies.

### A. Egr-1 Promoter

The Egr-1 promoter is defined herein as those 5' regulatory sequences necessary to control the DNA damaging agent-induced transcription of downstream sequences operably connected thereto. The Egr-1 promoter has complex structure which has previously been analyzed in the context of radiation- and H₂O₂-induced gene expression. It contains multiple ETS binding sites (ETS are transcriptional regulatory proteins), three of which exist as parts of two serum response elements (SRE's), SREI and SREII. The SRE's, also known as CArG motifs, are cis-elements that regulate the expression of many growth factor responsive genes. There are a total of six SRE's, each comprising the consensus CC(A+T-rich)6GG sequence.

The present inventors have previously demonstrated that a chimeric genetic construct consisting of the 5' Egr-1 CArG elements ligated to the TNF-α cDNA express high levels of intratumoral TNF-α following IR exposure of cells transduced with this construct. Tumors transduced with the chimeric Egr-TNF construct and treated with IR exhibited increased regression/cures compared with tumors treated with either agent alone, likely due to the intratumoral induction of TNF-α production by IR, and the cytotoxic interaction of TNF-α and IR on the tumor cells and the tumor vasculature (Weichselbaum *et al*., 2001; Staba *et al.,* 1998). In the present invention, the inventors used cisplatin, a commonly used chemotherapeutic agent that alters intracellular radical oxygen formation and damages DNA, to induce the TNF-α gene under control of the DNA damage / ROI inducible CArG elements of the Egr-1 promoter. The invention therefore provides the use of agents that cause DNA damage and/or produce ROI to induce Egr-1 and therefore to drive the expression of genes under the control of Egr-1 in expression vectors.

### B. DNA Damaging Chemicals

The term "DNA damaging chemical" refers to the any drug that induces, either directly or indirectly, damage to a DNA molecule. Of particular interest in the present invention are those drugs that generate free radicals. The following categories of chemicals are believed to effect DNA damage through one or more pathways.

### I. Alkylating Agents

alkylating agents are drugs that directly interact with genomic DNA to prevent the cancer cell from proliferating. This category of chemotherapeutic drugs represents agents that affect all phases of the cell cycle, that is, they are not phase-specific. Alkylating agents can be implemented to treat, for example, chronic leukemia, non-Hodgkin's lymphoma, Hodgkin's disease, multiple myeloma, and particular cancers of the breast, lung, and ovary. An alkylating agent, may include, but is not limited to, a nitrogen mustard, an ethylenimene, a methylmelamine, an alkyl sulfonate, a nitrosourea or a triazines.

They include but are not limited to: busulfan, chlorambucil, cisplatin, cyclophosphamide (cytoxan), dacarbazine, ifosfamide, mechlorethamine (mustargen), and melphalan. In specific aspects, troglitazaone can be used to treat cancer in combination with any one or more of these alkylating agents, some of which are discussed below.

### i. Nitrogen Mustards

A nitrogen mustard may be, but is not limited to, mechlorethamine (HN₂), which is used for Hodgkin's disease and non-Hodgkin's lymphomas; cyclophosphamide and/or ifosfamide, which are used in treating such cancers as acute or chronic lymphocytic leukemias, Hodgkin's disease, non-Hodgldn's lymphomas, multiple myeloma, neuroblastoma, breast, ovary, lung, Wilm's tumor, cervix testis and soft tissue sarcomas; melphalan (L-sarcolysin), which has been used to treat such cancers as multiple myeloma, breast and ovary; and chlorambucil, which has been used to treat diseases such as, for example, chronic lymphatic (lymphocytic) leukemia, malignant lymphomas including lymphosarcoma, giant follicular lymphoma, Hodgkin's disease and non-Hodgkin's lymphomas.

### a. Chlorambucil

Chlorambucil (also known as leukeran) is a bifunctional alkylating agent of the nitrogen mustard type that has been found active against selected human neoplastic diseases. Chlorambucil is known chemically as 4-[bis(2-chlorethyl)amino] benzenebutanoic acid.

Chlorambucil is available in tablet form for oral administration. It is rapidly and completely absorbed from the gastrointestinal tract. For example, after a single oral doses of about 0.6 mg/kg to about 1.2 mg/kg, peak plasma chlorambucil levels are reached within one hour and the terminal half-life of the parent drug is estimated at about 1.5 hours. About 0.1 mglkg/day to about 0.2 mg/kg/day or about 3 6mg/m²/day to about 6mg/m²/day or alternatively about 0.4 mg/kg may be used for antineoplastic treatment. Chlorambucil is not curative by itself but may produce clinically useful palliation.

### b. Cyclophosphamide

Cyclophosphamide is 2*H*-1,3,2-Oxazaphosphorin-2-amine, *N,N*-bis(2-chloroethyl)tetrahydro-, 2-oxide, monohydrate; termed Cytoxan available from Mead Johnson; and Neosar available from Adria. Cyclophosphamide is prepared by condensing 3-amino-1-propanol with *N,N*-bis(2-chlorethyl) phosphoramidic dichloride [(ClCH₂CH₂)₂N--POCl₂] in dioxane solution under the catalytic influence of triethylamine. The condensation is double, involving both the hydroxyl and the amino groups, thus effecting the cyclization.

Unlike other ß-chloroethylamino alkylators, it does not cyclize readily to the active ethyleneimonium form until activated by hepatic enzymes. Thus, the substance is stable in the gastrointestinal tract, tolerated well and effective by the oral and parental routes and does not cause local vesication, necrosis, phlebitis or even pain.

Suitable oral doses for adults include, for example, about 1 mg/kg/day to about 5 mglkg/day (usually in combination), depending upon gastrointestinal tolerance; or about 1 mg/kg/day to about 2 mg/kg/day; intravenous doses include, for example, initially about 40 mg/kg to about 50 mg/kg in divided doses over a period of about 2 days to about 5 days or about 10 mg/kg to about 15 mg/kg about every 7 days to about 10 days or about 3 mg/kg to about 5 mg/kg twice a week or about 1.5 mg/kg/day to about 3 mg/kg/day. In some aspects, a dose of about 250 mg/kg/day may be administered as an antineoplastic. Because of gastrointestinal adverse effects, the intravenous route is preferred for loading. During maintenance, a leukocyte count of about 3000/mm³ to 4000/mm³ usually is desired. The drug also sometimes is administered intramuscularly, by infiltration or into body cavities. It is available in dosage forms for injection of about 100 mg, about 200 mg and about 500 mg, and tablets of about 25 mg and about 50 mg.

### c. Melphalan

Melphalan, also known as alkeran, L-phenylalanine mustard, phenylalanine mustard, L-PAM, or L-sarcolysin, is a phenylalanine derivative of nitrogen mustard. Melphalan is a bifunctional alkylating agent which is active against selective human neoplastic diseases. It is known chemically as 4-[bis(2-chloroethyl)amino]-L-phenylalanine.

Melphalan is the active L-isomer of the compound and was first synthesized in 1953 by Bergel and Stock; the D-isomer, known as medphalan, is less active against certain animal tumors, and the dose needed to produce effects on chromosomes is larger than that required with the L-isomer. The racemic (DL-) form is known as merphalan or sarcolysin. Melphalan is insoluble in water and has a pKa₁ of about 2.1. Melphalan is available in tablet form for oral administration and has been used to treat multiple myeloma. Available evidence suggests that about one third to one half of the patients with multiple myeloma show a favorable response to oral administration of the drug.

Melphalan has been used in the treatment of epithelial ovarian carcinoma. One commonly employed regimen for the treatment of ovarian carcinoma has been to administer melphalan at a dose of about 0.2 mg/kg daily for five days as a single course. Courses are repeated about every four to five weeks depending upon hematologic tolerance (Smith and Rutledge, 1975; Young *et al*., 1978). Alternatively, in certain embodiments, the dose of melphalan used could be as low as about 0.05 mg/kg/day or as high as about 3 mg/kg/day or greater.

### ii. Ethylenimenes and Methymelamines

An ethylenimene and/or a methylmelamine include, but are not limited to, hexamethylmelamine, used to treat ovary cancer; and thiotepa, which has been used to treat bladder, breast and ovary cancer.

### iii. Alkyl Sulfonates

An alkyl sulfonate includes but is not limited to such drugs as busulfan, which has been used to treat chronic granulocytic leukemia. Busulfan (also known as myleran) is a bifunctional alkylating agent. Busulfan is known chemically as 1,4-butanediol dimethanesulfonate. Busulfan is available in tablet form for oral administration, wherein for example, each scored tablet contains about 2 mg busulfan and the inactive ingredients magnesium stearate and sodium chloride.

Busulfan is indicated for the palliative treatment of chronic myelogenous (myeloid, myelocytic, granulocytic) leukemia. Although not curative, busulfan reduces the total granulocyte mass, relieves symptoms of the disease, and improves the clinical state of the patient. Approximately 90% of adults with previously untreated chronic myelogenous leukemia will obtain hematologic remission with regression or stabilization of organomegaly following the ' use of busulfan. Busulfan has been shown to be superior to splenic irradiation with respect to survival times and maintenance of hemoglobin levels, and to be equivalent to irradiation at controlling splenomegaly.

### iv. Nitrosoureas

Nitrosureas, like alkylating agents, inhibit DNA repair proteins. They are used to treat non-Hodgkin's lymphomas, multiple myeloma, malignant melanoma, in addition to brain tumors.

A nitrosourea include but is not limited to a carmustine (BCNU), a lomustine (CCNL), a semustine (methyl-CCNU) or a streptozocin. Semustine has been used in such cancers as a primary brain tumor, a stomach or a colon cancer. Stroptozocin has been used to treat diseases such as a malignant pancreatic insulinoma or a malignant carcinoid. Streptozocin has been used to treat such cancers as a malignant melanoma, Hodgkin's disease and soft tissue sarcomas.

### a. Carmustine

Carmustine (sterile carmustine) is one of the nitrosoureas used in the treatment of certain neoplastic diseases. It is 1,3 bis (2-chloroethyl)-1-nitrosourea. It is lyophilized pale yellow flakes or congealed mass with a molecular weight of 214.06. It is highly soluble in alcohol and lipids, and poorly soluble in water. Carmustine is administered by intravenous infusion after reconstitution as recommended

Although it is generally agreed that carmustine alkylates DNA and RNA, it is not cross resistant with other alkylators. As with other nitrosoureas, it may also inhibit several key enzymatic processes by carbamoylation of amino acids in proteins.

Carmustine is indicated as palliative therapy as a single agent or in established combination therapy with other approved chemotherapeutic agents in brain tumors such as glioblastoma, brainstem glioma, medullobladyoma, astrocytoma, ependymoma, and metastatic brain tumors. Also it has been used in combination with prednisone to treat multiple myeloma. Carmustine has been used in treating such cancers as a multiple myeloma or a malignant melanoma. Carmustine has proved useful, in the treatment of Hodgkin's Disease and in non-Hodgkin's lymphomas, as secondary therapy in combination with other approved drugs in patients who relapse while being treated with primary therapy, or who fail to respond to primary therapy.

Sterile carmustine is commonly available in 100 mg single dose vials of lyophilized material. The recommended dose of carmustine as a single agent in previously untreated patients is about 150 mg/m² to about 200 mg/m² intravenously every 6 weeks. This may be given as a single dose or divided into daily injections such as about 75 mg/m² to about 100 mg/m² on 2 successive days. When carmustine is used in combination with other myelosuppressive drugs or in patients in whom bone marrow reserve is depleted, the doses should be adjusted accordingly. Doses subsequent to the initial dose should be adjusted according to the hematologic response of the patient to the preceding dose. It is of course understood that ether doses may be used in the present invention, for example about 10 mg/m², about 20 mg/m², about 30 mg/m², about 40 mg/m², about 50 mg/m², about 60 mg/m², about 70 mg/m², about 80 mg/m², about 90 mg/m² to about 100 mg/m².

### b: Lomustine

Lomustine is one of the nitrosoureas used in the treatment of certain neoplastic diseases. It is 1-(2-chloro-ethyl)-3-cyclohexyl-1 nitrosourea. It is a yellow powder with the empirical formula of C₉H₁₆ClN₃O₂ and a molecular weight of 233.71. Lomustine is soluble in 10% ethanol (about 0.05 mg/mL) and in absolute alcohol (about 70 mg/mL). Lomustine is relatively insoluble in water (less than about 0.05 mg/mL). It is relatively unionized at a physiological pH. Inactive ingredients in lomustine capsules are: magnesium stearate and mannitol.

Although it is generally agreed that lomustine alkylates DNA and RNA, it is not cross resistant with other alkylators. As with other nitrosoureas, it may also inhibit several key enzymatic processes by carbamoylation of amino acids in proteins.

Lomustine may be given orally. Following oral administration of radioactive lomustine at doses ranging from about 30 mg/m² to 100 mg/m², about half of the radioactivity given was excreted in the form of degradation products within 24 hours. The serum half-life of the metabolites ranges from about 16 hours to about 2 days. Tissue levels are comparable to plasma levels at 15 minutes after intravenous administration.

Lomustine has been shown to be useful as a single agent in addition to other treatment modalities, or in established combination therapy with other approved chemotherapeutic agents in both primary and metastatic brain tumors, in patients who have already received appropriate surgical and/or radiotherapeutic procedures. Lomustine has been used to treat such cancers as small-cell lung cancer. It has also proved effective in secondary therapy against Hodgkin's Disease in combination with other approved drugs in patients who relapse while being treated with primary therapy, or who fail to respond to primary therapy.

The recommended dose of lomustine in adults and children as a single agent in previously untreated patients is about 130 mg/m² as a single oral dose every 6 weeks. In individuals with compromised bone marrow function, the dose should be reduced to about 100 mg/m² every 6 weeks. When lomustine is used in combination with other myelosuppressive drugs, the doses should be adjusted accordingly. It is understood that other doses may be used for example, about 20 mg/m², about 30mg/m², about 40 mg/m², about 50 mg/m², about 60 mg/m², about 70 mg/m², about 80 mg/m², about 90 mg/m², about 100 mg/m² to about 120 mg/m².

### c. Triazine

A triazine include but is not limited to such drugs as a dacabazine (DTIC; dimethyltriazenoimidaz olecarboxamide), used in the treatment of such cancers as a malignant melanoma, Hodgkin's disease and a soft-tissue sarcoma.

### II. Antimetabolites

Antimetabolites disrupt DNA and RNA synthesis. Unalike alkylating agents, they specifically influence the cell cycle during S phase. They have used to combat chronic leukemias in addition to tumors of breast, ovary and the gastrointestinal tract. Antimetabolites can be differentiated into various categories, such as folic acid analogs, pyrimidine analogs and purine analogs and related inhibitory compounds. Antimetabolites include but are not limited to, 5-fluorouracil (5-FU), cytarabine (Ara-C), fludarabine, gemcitabine, and methotrexate.

### i. Folic Acid Analogs

Folic acid analogs include but are not limited to compounds such as methotrexate (amethopterin), which has been used in the treatment of cancers such as acute lymphocytic leukemia, choriocarcinoma, mycosis fungoides, breast, head and neck, lung and osteogenic sarcoma.

### ii. Pyrimidine Analogs

Pyrimidine analogs include such compounds as cytarabine (cytosine arabinoside), 5-fluorouracil (fluouracil; 5-FU) and floxuridine (fluorode-oxyuridine; FudR). Cytarabine has been used in the treatment of cancers such as acute granulocytic leukemia and acute lymphocytic leukemias. Floxuridine and 5-fluorouracil have been used in the treatment of cancers such as breast, colon, stomach, pancreas, ovary, head and neck, urinary bladder and topical premalignant skin lesions.

5-Fluorouracil (5-FU) has the chemical name of 5-fluoro-2,4(1H,3H)-pyrimidinedione. Its mechanism of action is thought to be by blocking the methylation reaction of deoxyuridylic acid to thymidylic acid. Thus, 5-FU interferes with the synthesis of deoxyribonucleic acid (DNA) and to a lesser extent inhibits the formation of ribonucleic acid (RNA). Since DNA and RNA are essential for cell division and proliferation, it is thought that the effect of 5-FU is to create a thymidine deficiency leading to cell death. Thus, the effect of 5-FU is found in cells that rapidly divide, a characteristic of metastatic cancers.

### iii. Purine Analogs and Related Inhibitors

Purine analogs and related compounds include, but are not limited to, mercaptopurine (6-mercaptopurine; 6-MP), thioguanine (6-thioguanine; TG) and pentostatin (2-deoxycoformycin). Mercaptopurine has been used in acute lymphocytic, acute granulocytic and chronic granulocytic leukemias. Thrioguanine has been used in the treatment of such cancers as acute granulocytic leukemia, acute lymphocytic leukemia and chronic lymphocytic leukemia. Pentostatin has been used in such cancers as hairy cell leukemias, mycosis fungoides and chronic lymphocytic leukemia.

### III. Natural Products

Natural products generally refer to compounds originally isolated from a natural source, and identified has having a pharmacological activity. Such compounds, analogs and derivatives thereof may be, isolated from a natural source, chemically synthesized or recombinantly produced by any technique known to those of skill in the art. Natural products include such categories as mitotic inhibitors, antitumor antibiotics, enzymes and biological response modifiers.

### i. Mitotic Inhibitors

Mitotic inhibitors include plant alkaloids and other natural agents that can inhibit either protein synthesis required for cell division or mitosis. They operate during a specific phase during the cell cycle. Mitotic inhibitors include, for example, docetaxel, etoposide (VP16), teniposide, paclitaxel, taxol, vinblastine, vincristine, and vinorelbine.

### a. Epipodophyllotoxins

Epipodophyllotoxins include such compounds as teniposide and VP16. VP16 is also known as etoposide and is used primarily for treatment of testicular tumors, in combination with bleomycin and cisplatin, and in combination with cisplatin for small-cell carcinoma of the lung. Teniposide and VP16 are also active against cancers such as testis, other lung cancer, Hodgkin's disease, non-Hodgkin's lymphomas, acute granulocytic leukemia, acute nonlymphocytic leukemia, carcinoma of the breast, and Kaposi's sarcoma associated with acquired immunodeficiency syndrome (AIDS).

VP16 is available as a solution (*e.g*., 20 mg/ml) for intravenous administration and as 50 mg, liquid-filled capsules for oral use. For small-cell carcinoma of the lung, the intravenous dose (in combination therapy) is can be as much as about 100 mg/m² or as little as about 2 mg/ m², routinely about 35 mg/m², daily for about 4 days, to about 50 mg/m², daily for about 5 days have also been used. When given orally, the dose should be doubled. Hence the doses for small cell lung carcinoma may be as high as about 200 mg/m² to about 250 mg/m². The intravenous dose for testicular cancer (in combination therapy) is about 50 mg/m² to about 100 mg/m² daily for about 5 days, or about 100 mg/m² on alternate days, for three doses. Cycles of therapy are usually repeated about every 3 to 4 weeks. The drug should be administered slowly (*e.g*., about 30 minutes to about 60 minutes) as an infusion in order to avoid hypotension and bronchospasm, which are probably due to the solvents used in the formulation.

### b. Taxoids

Taxoids are a class of related compounds isolated from the bark of the ash tree, *Taxus brevifolia.* Taxoids include but are not limited to compounds such as docetaxel and paclitaxel.

Paclitaxel binds to tubulin (at a site distinct from that used by the vinca alkaloids) and promotes the assembly of microtubules. Paclitaxel is being evaluated clinically; it has activity against malignant melanoma and carcinoma of the ovary. In certain aspects, maximal doses are about 30 mg/m² per day for about 5 days or about 210 mg/m² to about 250 mg/m² given once about every 3 weeks.

### c. Vinca Alkaloids

Vinca alkaloids are a type of plant alkaloid identified to have pharmaceutical activity. They include such compounds as vinblastine (VLB) and vincristine.

### 1. Vinblastine

Vinblastine is an example of a plant alkaloid that can be used for the treatment of cancer and precancer. When cells are incubated with vinblastine, dissolution of the microtubules occurs.

Unpredictable absorption has been reported after oral administration of vinblastine or vincristine. At the usual clinical doses the peak concentration of each drug in plasma is approximately 0.4 mM. Vinblastine and vincristine bind to plasma proteins. They are extensively concentrated in platelets and to a lesser extent in leukocytes and erythrocytes.

After intravenous injection, vinblastine has a multiphasic pattern of clearance from the plasma; after distribution, drug disappears from plasma with half-lives of approximately 1 and 20 hours. Vinblastine is metabolized in the liver to biologically activate derivative desacetylvinblastine. Approximately 15% of an administered dose is detected intact in the urine, and about 10% is recovered in the feces after biliary excretion. Doses should be reduced in patients with hepatic dysfunction. At least a 50% reduction in dosage is indicated if the concentration of bilirubin in plasma is greater than 3 mg/dl (about 50 mM).

Vinblastine sulfate is available in preparations for injection. When the drug is given intravenously; special precautions must be taken against subcutaneous extravasation, since this may cause painful irritation and ulceration. The drug should not be injected into an extremity with impaired circulation. After a single dose of 0.3 mg/kg of body weight, myelosuppression reaches its maximum in about 7 days to about 10 days. If a moderate level of leukopenia (approximately 3000 cells/mm³) is not attained, the weekly dose may be increased gradually by increments of about 0.05 mg/kg of body weight. In regimens designed to cure testicular cancer, vinblastine is used in doses of about 0.3 mg/kg about every 3 weeks irrespective of blood cell counts or toxicity.

An important clinical use of vinblastine is with bleomycin and cisplatin in the curative therapy of metastatic testicular tumors. Beneficial responses have been reported in various lymphomas, particularly Hodgkin's disease, where significant improvement may be noted in 50 to 90% of cases. The effectiveness of vinblastine in a high proportion of lymphomas is not diminished when the disease is refractory to alkylating agents. It is also active in Kaposi's sarcoma, testis cancer, neuroblastoma, and Letterer-Siwe disease (histiocytosis X), as well as in carcinoma of the breast and choriocarcinoma in women.

Doses of about 0.1 mg/kg to about 0.3 mg/kg can be administered or about 1.5 mg/m² to about 2 mg/m² can also be administered. Alternatively, about 0.1 mg/m², about 0.12 mg/m², about 0.14 mg/m², about 0.15 mg/m², about 0.2 mg/m², about 0.25 mg/m², about 0.5 mg/m², about 1.0 mg/m², about 1.2 mg/m², about 1.4 mg/m², about 1.5 mg/m², about 2.0 mg/m², about 2.5 mg/m², about 5.0 mg/m², about 6 mg/m², about 8 mg/m², about 9 mg/m², about 10 mg/m²*,* to about 20 mg/m², can be given.

### 2. Vincristine

Vincristine blocks mitosis and produces metaphase arrest. It seems likely that most of the biological activities of this drug can be explained by its ability to bind specifically to tubulin and to block the ability of protein to polymerize into microtubules. Through disruption of the microtubules of the mitotic apparatus, cell division is arrested in metaphase. The inability to segregate chromosomes correctly during mitosis presumably leads to cell death.

The relatively low toxicity of vincristine for normal marrow cells and epithelial cells make this agent unusual among anti-neoplastic drugs, and it is often included in combination with other myelosuppressive agents. Unpredictable absorption has been reported after oral administration of vinblastine or vincristine. At the usual clinical doses the peak concentration of each drug in plasma is about 0.4 mM.

Vinblastine and vincristine bind to plasma proteins. They are extensively concentrated in platelets and to a lesser extent in leukocytes and erythrocytes. Vincristine has a multiphasic pattern of clearance from the plasma; the terminal half-life is about 24 hours. The drug is metabolized in the liver, but no biologically active derivatives have been identified. Doses should be reduced in patients with hepatic dysfunction. At least a 50% reduction in dosage is indicated if the concentration of bilirubin in plasma is greater than about 3 mg/dl (about 50 mM).

Vincristine sulfate is available as a solution (e.g., 1 mg/ml) for intravenous injection. Vincristine used together with corticosteroids is presently the treatment of choice to induce remissions in childhood leukemia; the optimal dosages for these drugs appear to be vincristine, intravenously, about 2 mg/m² of body-surface area, weekly; and prednisone, orally, about 40 mg/m², daily. Adult patients with Hodgkin's disease or non-Hodgkin's lymphomas usually receive vincristine as a part of a complex protocol. When used in the MOPP regimen, the recommended dose of vincristine is about 1.4 mg/m². High doses of vincristine seem to be tolerated better by children with leukemia than by adults, who may experience sever neurological toxicity. Administration of the drug more frequently than every 7 days or at higher doses seems to increase the toxic manifestations without proportional improvement in the response rate. Precautions should also be used to avoid extravasation during intravenous administration of vincristine. Vincristine (and vinblastine) can be infused into the arterial blood supply of tumors in doses several times larger than those that can be administered intravenously with comparable toxicity.

Vincristine has been effective in Hodgkin's disease and other lymphomas. Although it appears to be somewhat less beneficial than vinblastine when used alone in Hodgkin's disease, when used with mechlorethamine, prednisone, and procarbazine (the so-called MOPP regimen), it is the preferred treatment for the advanced stages (III and IV) of this disease. In non-Hodgkin's lymphomas, vincristine is an important agent, particularly when used with cyclophosphamide, bleomycin, doxorubicin, and prednisone. Vincristine is more useful than vinblastine in lymphocytic leukemia. Beneficial response have been reported in patients with a variety of other neoplasms, particularly Wilms' tumor, neuroblastoma, brain tumors, rhabdomyosarcoma, small cell lung, and carcinomas of the breast, bladder, and the male and female reproductive systems.

Doses of vincristine include about 0.01 mg/kg to about 0.03 mg/kg or about 0.4 mg/m² to about 1.4 mg/m² can be administered or about 1.5 mg/m² to about 2 mg/m² can also be administered. Alternatively, in certain embodiments, about 0.02 mg/m², about 0.05 mg/m², about 0.06 mg/m², about 0.07 mg/m², about 0.08 mg/m², about 0.1 mg/m², about 0.12 mg/m², about 0.14 mg/m², about 0.15 mg/m², about 0.2 mg/m², about 0.25 mg/m² can be given as a constant intravenous infusion.

### d. Antitumor Antibiotics

Antitumor antibiotics have both antimicrobial and cytotoxic activity. These drugs also interfere with DNA by chemically inhibiting enzymes and mitosis or altering cellular membranes. These agents are not phase specific so they work in all phases of the cell cycle. Thus, they are widely used for a variety of cancers. Examples of antitumor antibiotics include, but are not limited to, bleomycin, dactinomycin, and plicamycin (mithramycin). Widely used in clinical setting for the treatment of neoplasms these compounds generally are administered through intravenous bolus injections or orally.

### 1. Mitomycin

Mitomycin (also known as mutamycin and/or mitomycin-C) is an antibiotic isolated from the broth of *Streptomyces caespitosus* which has been shown to have antitumor activity. The compound is heat stable, has a high melting point, and is freely soluble in organic solvents.

Mitomycin selectively inhibits the synthesis of deoxyribonucleic acid (DNA). The guanine and cytosine content correlates with the degree of mitomycin-induced cross-linking. At high concentrations of the drug, cellular RNA and protein synthesis are also suppressed. Mitomycin has been used in tumors such as stomach, cervix, colon, breast, pancreas, bladder and head and neck.

In humans, mitomycin is rapidly cleared from the serum after intravenous administration. Time required to reduce the serum concentration by about 50% after a 30 mg. bolus injection is 17 minutes. After injection of 30 mg, 20 mg, or 10 mg I.V., the maximal serum concentrations were 2.4 mg/mL, 1.7 mg/mL, and 0.52 mg/mL, respectively. Clearance is effected primarily by metabolism in the liver, but metabolism occurs in other tissues as well. The rate of clearance is inversely proportional to the maximal serum concentration because, it is thought, of saturation of the degradative pathways. Approximately 10% of a dose of mitomycin is excreted unchanged in the urine. Since metabolic pathways are saturated at relatively low doses, the percent of a dose excreted in urine increases with increasing dose. In children, excretion of intravenously administered mitomycin is similar.

### 2. Actinomycin D

Actinomycin D (Dactinomycin) [50-76-0]; C₆₂H₈₆N₁₂O₁₆ (1255.43) is an antineoplastic drug that inhibits DNA-dependent RNA polymerase. It is often a component of first-choice combinations for treatment of diseases such as, for example, choriocarcinoma, embryonal rhabdomyosarcoma, testicular tumor, Kaposi's sarcoma and Wilms' tumor. Tumors that fail to respond to systemic treatment sometimes respond to local perfusion. Dactinomycin potentiates radiotherapy. It is a secondary (efferent) immunosuppressive.

In certain specific aspects, actinomycin D is used in combination with agents such as, for example, primary surgery, radiotherapy, and other drugs, particularly vincristine and cyclophosphamide. Antineoplastic activity has also been noted in Ewing's tumor, Kaposi's sarcoma, and soft-tissue sarcomas. Dactinomycin can be effective in women with advanced cases of choriocarcinoma. It also produces consistent responses in combination with chlorambucil and methotrexate in patients with metastatic testicular carcinomas. A response may sometimes be observed in patients with Hodgkin's disease and non-Hodgkin's lymphomas. Dactinomycin has also been used to inhibit immunological responses, particularly the rejection of renal transplants.

Half of the dose is excreted intact into the bile and 10% into the urine; the half-life is about 36 hours. The drug does not pass the blood-brain barrier. Actinomycin D is supplied as a lyophilized powder (0/5 mg in each vial). The usual daily dose is about 10 mg/kg to about 15 mg/kg; this is given intravenously for about 5 days; if no manifestations of toxicity are encountered, additional courses may be given at intervals of about 3 weeks to about 4 weeks. Daily injections of about 100 mg to about 400 mg have been given to children for about 10 days to about 14 days; in other regimens, about 3 mg/kg to about 6 mg/kg, for a total of about 125 mg/kg, and weekly maintenance doses of about 7.5 mg/kg have been used. Although it is safer to administer the drug into the tubing of an intravenous infusion, direct intravenous injections have been given, with the precaution of discarding the needle used to withdraw the drug from the vial in order to avoid subcutaneous reaction. Exemplary doses may be about 100 mg/m², about 150 mg/m², about 175 mg/m², about 200 mg/m², about 225 mg/m², about 250 mg/m², about 275 mg/m², about 300 mg/m², about 350 mg/m², about 400 mg/m², about 425 mg/m², about 450 mg/m², about 475 mg/m², to about 500 mg/m².

### 3. Bleomycin

Bleomycin is a mixture of cytotoxic glycopeptide antibiotics isolated from a strain of *Streptomyces verticillus.* Although the exact mechanism of action of bleomycin is unknown, available evidence would seem to indicate that the main mode of action is the inhibition of DNA synthesis with some evidence of lesser inhibition of RNA and protein synthesis.

In mice, high concentrations of bleomycin are found in the skin, lungs, kidneys, peritoneum, and lymphatics. Tumor cells of the skin and lungs have been found to have high concentrations of bleomycin in contrast to the low concentrations found in hematopoietic tissue. The low concentrations of bleomycin found in bone marrow may be related to high levels of bleomycin degradative enzymes found in that tissue.

In patients with a creatinine clearance of greater than about 35 mL per minute, the serum or plasma terminal elimination half-life of bleomycin is approximately 115 minutes. In patients with a creatinine clearance of less than about 35 mL per minute, the plasma or serum terminal elimination half-life increases exponentially as the creatinine clearance decreases. In humans, about 60% to about 70% of an administered dose is recovered in the urine as active bleomycin. In specific embodiments, bleomycin may be given by the intramuscular, intravenous, or subcutaneous routes. It is freely soluble in water. Because of the possibility of an anaphylactoid reaction, lymphoma patients should be treated with two units or less for the first two doses. If no acute reaction occurs, then the regular dosage schedule may be followed.

In preferred aspects, bleomycin should be considered a palliative treatment. It has been shown to be useful in the management of the following neoplasms either as a single agent or in proven combinations with other approved chemotherapeutic agents in squamous cell carcinoma such as head and neck (including mouth, tongue, tonsil, nasopharynx, oropharynx, sinus, palate, lip, buccal mucosa, gingiva, epiglottis, larynx), esophagus, lung and genitourinary tract, Hodgkin's disease, non-Hodgkin's lymphoma, skin, penis, cervix, and vulva. It has also been used in the treatment of lymphomas and testicular carcinoma.

Improvement of Hodgkin's Disease and testicular tumors is prompt and noted within 2 weeks. If no improvement is seen by this time, improvement is unlikely. Squamous cell cancers respond more slowly, sometimes requiring as long as 3 weeks before any improvement is noted.

### IV. Miscellaneous Agents

Some chemotherapy agents do not qualify into the previous categories based on their activities. They include, but are not limited to, platinum coordination complexes, anthracenedione, substituted urea, methyl hydrazine derivative, adrenalcortical suppressant, amsacrine, L-asparaginase, and tretinoin. It is contemplated that they are included within the compositions and uses of the present invention for use in combination therapies.

### i. Platinum Coordination Complexes

Platinum coordination complexes include such compounds as carboplatin and cisplatin (cis-DDP). Cisplatin has been widely used to treat cancers such as, for example, metastatic testicular or ovarian carcinoma, advanced bladder cancer, head or neck cancer, cervical cancer, lung cancer or other tumors. Cisplatin is not absorbed orally and must therefore be delivered via other routes, such as for example, intravenous, subcutaneous, intratumoral or intraperitoneal injection. Cisplatin can be used alone or in combination with other agents, with efficacious doses used in clinical applications of about 15 mg/m² to about 20 mg/m² for 5 days every three weeks for a total of three courses being contemplated in certain embodiments. Doses may be, for example, about 0.50 mg/m², about 1.0 mg/m², about 1.50 mg/m², about 1.75 mg/m², about 2.0 mg/m², about 3.0 mg/m², about 4.0 mg/m², about 5.0 mg/m², to about 10 mg/m².

The present inventors have found that cisplatin, which stimulates ROI production, induces the CArG elements of the Egr-1 promoter. For example, cisplatin induced the production of TNF-α in human and rodent cancer cells infected with an adenoviral vector encoding the CArG elements of the Egr-1 promoter ligated upstream to a cDNA encoding TNF-α. Thus, the present invention provides a new approach that combines the use of, chemotherapeutic agents that can produce ROI or DNA damage, such as cisplatin, with the temporal and spatial control of gene therapy using antitumor genes.

### ii. Other Agents

Anthracenediones, such as mitoxantrone, have been used for treating acute granulocytic leukemia and breast cancer. A substituted urea such as hydroxyurea has been used in treating chronic granulocytic leukemia, polycythemia vera, essental thrombocytosis and malignant melanoma. A methyl hydrazine derivative such as procarbazine (N-methylhydrazine, MIH) has been used in the treatment of Hodgkin's disease. An adrenocortical suppressant such as mitotane has been used to treat adrenal cortex cancer, while aminoglutethimide has been used to treat Hodgkin's disease.

### V. Doses

Doses for DNA damaging agents are well known to those of skill in the art (see for example, the "Physicians Desk Reference", Goodman & Gilman's "The Pharmacological Basis of Therapeutics", "Remington's Pharmaceutical Sciences", and "The Merck Index, Eleventh Edition," incorporated herein by reference in relevant parts), and may be combined with the invention in light of the disclosures herein. Some variation in dosage will necessarily occur depending on the condition of the subject being treated. The person responsible for administration will, in any event, determine the appropriate dose for the individual subject. Examples of specific chemotherapeutic agents and dose regimes are also described herein. Of course, all of these dosages and agents described herein are exemplary rather than limiting, and other doses or agents may be used by a skilled artisan for a specific patient or application. Any dosage in-between these points, or range derivable therein is also expected to be of use in the invention.

### C. Therapeutic Genes

### I. Tumor Suppressors

p53 currently is recognized as a tumor suppressor gene. High levels of mutant p53 have been found in many cells transformed by chemical carcinogenesis, ultraviolet radiation, and several viruses. The p53 gene is a frequent target of mutational inactivation in a wide variety of human tumors and is already documented to be the most frequently-mutated gene in common human cancers. It is mutated in over 50% of human NSCLC (Hollstein *et al.,* 1991) and in a wide spectrum of other tumors.

The p53 gene encodes a 393-amino acid phosphoprotein that can form complexes with host proteins such as SV40 large-T antigen and adenoviral E1B. The protein is found in normal tissues and cells, but at concentrations which are minute by comparison with transformed cells or tumor tissue. Interestingly, wild-type p53 appears to be important in regulating cell growth and division. Overexpression of wild-type p53 has been shown in some cases to be anti-proliferative in human tumor cell lines. Thus, p53 can act as a negative regulator of cell growth (Weinberg, 1991) and may directly suppress uncontrolled cell growth or indirectly activate genes that suppress this growth. Thus, absence or inactivation of wild-type p53 may contribute to transformation. However, some studies indicate that the presence of mutant p53 may be necessary for full expression of the transforming potential of the gene.

Wild-type p53 is recognized as an important growth regulator in many cell types. Missense mutations are common for the p53 gene and are essential for the transforming ability of the oncogene. A single genetic change prompted by point mutations can create carcinogenic p53, in as much as mutations in p53 are known to abrogate the tumor suppressor capability of wild-type p53. Unlike other oncogenes, however, p53 point mutations are known to occur in at least 30 distinct codons, often creating dominant alleles that produce shifts in cell phenotype without a reduction to homozygosity. Additionally, many of these dominant negative alleles appear to be tolerated in the organism and passed on in the germ line. Various mutant alleles appear to range from minimally dysfunctional to strongly penetrant, dominant negative alleles (Weinberg, 1991).

Casey and colleagues have reported that transfection of DNA encoding wild-type p53 into two human breast cancer cell lines restores growth suppression control in such cells (Casey *et al.,* 1991). A similar effect also has been demonstrated on transfection of wild-type, but not mutant, p53 into human lung cancer cell lines (Takahasi *et al.,* 1992). p53 appears dominant over the mutant gene and will select against proliferation when transfected into cells with the mutant gene. Normal expression of the transfected p53 does not affect the growth of normal or non-malignant cells with endogenous p53. Thus, such constructs might be taken up by normal cells without adverse effects. It is thus proposed that the treatment of p53-associated cancers with wild-type p53 will reduce the number of malignant cells or their growth rate.

The major transitions of the eukaryotic cell cycle are triggered by cyclin-dependent kinases, or CDK's. One CDK, cyclin-dependent kinase 4 (CDK4), regulates progression through the G₁. The activity of this enzyme may be to phosphorylate Rb at late G₁. The activity of CDK4 is controlled by an activating subunit, D-type cyclin, and by an inhibitory subunit p16^{INK4}. The p16^{INK4} has been biochemically characterized as a protein that specifically binds to and inhibits CDK4, and thus may regulate Rb phosphorylation (Serrano *et al.,* 1993; Serrano *et al.*, 1995). Since the p16^{INK4} protein is a CDK4 inhibitor (Serrano, 1993), deletion of this gene may increase the activity of CDK4, resulting in hyperphosphorylation of the Rb protein. p16: also is known to regulate the function of CDK6.

p16^{INK4} belongs to a newly described class of CDK-inhibitory proteins that also includes p15 ^{INK4B}, p21^{WAF1}, and p27^{KIP1}. The p16^{INK4} gene maps to 9p21, a chromosome region frequently deleted in many tumor types. Homozygous deletions and mutations of the p16^{INK4} gene are frequent in human tumor cell lines. This evidence suggests that the p16^{I1K4} gene is a tumor suppressor gene. This interpretation has been challenged, however, by the observation that the frequency of the p16^{INK4} gene alterations is much lower in primary uncultured tumors than in cultured cell lines (Caldas *et al.,* 1994; Cheng *et al.,* 1994; Hussussian *et al*., 1994; Kamb *et al.,* 1994; Kamb *et al.,* 1994; Mori *et al.,* 1994; Okamoto *et al.,* 1994; Nobori *et al.,* 1995; Orlow *et al.,* 1994; Arap *et al.,* 1995). However, it was later shown that while the p16 gene was intact in many primary tumors, there were other mechanisms that prevented p16 protein expression in a large percentage of some tumor types. p16 promoter hypermethylation is one of these mechanisms (Merlo *et al*., 1995; Herman, 1995; Gonzalez-Zulueta, 1995). Restoration of wild-type p16^{INKa} function by transfection with a plasmid expression vector reduced colony formation by some human cancer cell lines (Okamoto, 1994; Arap, 1995). Delivery of p16 with adenovirus vectors inhibits proliferation of some human cancer lines and reduces the growth of human tumor xenografts.

C-CAM is expressed in virtually all epithelial cells (Odin and Obrink, 1987). C-CAM, with an apparent molecular weight of 105 kD, was originally isolated from the plasma membrane of the rat hepatocyte by its reaction with specific antibodies that neutralize cell aggregation (Obrink, 1991). Recent studies indicate that, structurally, C-CAM belongs to the immunoglobulin (Ig) superfamily and its sequence is highly homologous to carcinoembryonic antigen (CEA) (Lin and Guidotti, 1989). Using a baculovirus expression system, Cheung *et al.* (1993) demonstrated that the first Ig domain of C-CAM is critical for cell adhesive activity.

Cell adhesion molecules, or CAM's are known to be involved in a complex network of molecular interactions that regulate organ development and cell differentiation (Edelman, 1985). Recent data indicate that aberrant expression of CAM's maybe involved in the tumorigenesis of several neoplasms; for example, decreased expression of E-cadherin, which is predominantly expressed in epithelial cells, is associated with the progression of several kinds of neoplasms (Edelman and Crossin, 1991; Frixen *et al.,* 1991; Bussemakers *et al.,* 1992; Matsura *et al.,* 1992; Umbas *et al.,* 1992). Also, Giancotti and Ruoslahti (1990) demonstrated that increasing expression of α₅β₁ integrin by gene transfer can reduce tumorigenicity of Chinese hamster ovary cellos *in vivo*. C-CAM now has been shown to suppress tumor growth *in vitro* and *in vivo.*

Other tumor suppressors that may be employed according to the present invention include p21, p15, BRCA1, BRCA2, IRF-1, PTEN, RB, APC, DCC, NF-1, NF-2, WT-1, MEN-I, MEN-II, zac1, p73, VHL, FCC, MCC, DBCCR1, DCP4 and p57.

### II. Inducers of Apoptosis

Inducers of apoptosis, such as Bax, Bak, Bcl-Xg, Bad, Bim, Bik, Bid, Harakiri, Ad E1B, Bad, ICE-CED3 proteases, TRAIL, SARP-2 and apoptin, similarly could find use according to the present invention. In addition, the delivery and regulated expression of cytotoxic genes have been described in the U.S. Patent Application entitled, "Induction of Apoptic or Cytotoxic Gene Expression by Adenoviral Mediated Gene Codelivery," filed March 11, 1999 (specifically incorporated herein by reference).

### III. Enzymes

Various enzyme genes are of interest according to the present invention. Such enzymes include cytosine deaminase, adenosine deaminase, hypoxanthine-guanine phosphoribosyltransferase, and human thymidine kinase.

### IV. Cytokines, Hormones and Growth Factors

Another class of genes that is contemplated to be inserted into the vectors of the present invention include interleukins and cytokines. Interleukin 1, IL-2, IL-3, IL-4, IL-5, IL-6, IL-7, IL-8, IL-9, IL-10, IL-11, IL-12, IL-13, IL-14, IL-15, β-interferon, α-interferon, γ-interferon, angiostatin, thrombospondin, endostatin, METH-1, METH-2, GM-CSF, G-CSF, M-CSF and tumor necrosis factor (TNF).

TNF-α is a cytokine secreted by macrophages and other hematopoetic cells that has antitumor activity in animal studies (Old, 1985; Fiers, 1991). TNF-α is cytotoxic for many malignant cells and also plays an important role in the defense against viral, bacterial and parasitic infections and in autoimmune responses (Fiers, 1991). A direct toxic effect on tumor cells, as well as cytotoxic and thrombotic effects on the tumor vasculature, mediate the antitumor effects of TNF-α (Watanabe *et al.,* 1988; Tartaglia *et al.,* 1993; Robaye *et al.,* 1991; Havell *et al*., 1988; Obrador *et al.,* 2001; Slungaard *et al.,* 1990; Mauceri *et al.,* 2002). The combination of TNF-α with chemotherapeutic agents, such as cisplatin and adriamycin, that damage DNA has demonstrated synergistic effects in experimental models (Duan *et al.,* 2001; Bonavida *et al.,* 1990). Recently, isolated limb perfusion with melphalan, a bi-functional alkylating agent, and TNF-α has been reported to be a successful therapeutic strategy for limb sarcomas and melanomas (Thom *et al.,* 1995) However, systemic toxicities have limited the use of TNF-α in human cancer therapy (Spriggs *et al.,* 1988).

The present invention provides the chemo-induction of TNF-α under the control of the inducible Egr-1 promoter, which can be induced by ROI's, damaged DNA and IR, by a chemotherapeutic agent according to claim 1. Studies in mice models of cancer and human cancer cells show that the chemo-induction of TNF-α in itself did not cause any toxicity.

### V. Toxins

Various toxins are also contemplated to be useful as part of the expression vectors of the present invention, these toxins include bacterial toxins such as ricin A-chain (Burbage, 1997), diphtheria toxin A (Massuda *et al.,* 1997; Lidor, 1997), pertussis toxin A subunit, *E. coli* enterotoxin toxin A subunit, cholera toxin A subunit and pseudomonas toxin c-terminal. Recently, it was demonstrated that transfection of a plasmid containing the fusion protein regulatable diphtheria toxin A chain gene was cytotoxic for cancer cells. Thus, gene transfer of regulated toxin genes might also be applied to the treatment of cancers (Massuda *et al.,* 1997).

### VI. Antisense Constructs

Antisense methodology takes advantage of the fact that nucleic acids tend to pair with "complementary" sequences. By complementary, it is meant that polynucleotides are those which are capable of base-pairing according to the standard Watson-Crick complementarity rules. That is, the larger purines will base pair with the smaller pyrimidines to form combinations of guanine paired with cytosine (G:C) and adenine paired with either thymine (A:T) in the case of DNA, or adenine paired with uracil (A:U) in the case of RNA. Inclusion of less common bases such as inosine, 5-methylcytosine, 6-methyladenine, hypoxanthine and others in hybridizing sequences does not interfere with pairing.

Targeting double-stranded (ds) DNA with polynucleotides leads to triple-helix formation; targeting RNA will lead to double-helix formation. Antisense polynucleotides, when introduced into a target cell, specifically bind to their target polynucleotide and interfere with transcription, RNA processing, transport, translation and/or stability. Antisense RNA constructs, or DNA encoding such antisense RNA's, may be employed to inhibit gene transcription or translation or both within a host cell, either *in vitro* or *in vivo,* such as within a host animal, including a human subject.

Antisense constructs may be designed to bind to the promoter and other control regions, exons, introns or even exon-intron boundaries of a gene. It is contemplated that the most effective antisense constructs will include regions complementary to intron/exon splice junctions. Thus, it is proposed that a preferred embodiment includes an antisense construct with complementarity to regions within 50-200 bases of an intron-exon splice junction. It has been observed that some exon sequences can be included in the construct without seriously affecting the target selectivity thereof. The amount of exonic material included will vary depending on the particular exon and intron sequences used. One can readily test whether too much exon DNA is included simply by testing the constructs *in vitro* to determine whether normal cellular function is affected or whether the expression of related genes having complementary sequences is affected.

As stated above, "complementary" or "antisense" means polynucleotide sequences that are substantially complementary over their entire length and have very few base mismatches. For example, sequences of fifteen bases in length may be termed complementary when they have complementary nucleotides at thirteen or fourteen positions. Naturally, sequences which are completely complementary will be sequences which are entirely complementary throughout their entire length and have no base mismatches. Other sequences with lower degrees of homology also are contemplated. For example, an antisense construct which has limited regions of high homology, but also contains a non-homologous region (e.g., ribozyme; see below) could be designed. These molecules, though having less than 50% homology, would bind to target sequences under appropriate conditions.

It may be advantageous to combine portions of genomic DNA with cDNA or synthetic sequences to generate specific constructs. For example, where an intron is desired in the ultimate construct, a genomic clone will need to be used. The cDNA or a synthesized polynucleotide may provide more convenient restriction sites for the remaining portion of the construct and, therefore, would be used for the rest of the sequence.

Particular oncogenes that are targets for antisense constructs are *ras, myc, neu, raf, erb, src, fms, jun, trk, ret, hst, gsp, bcl-2* and *abl.* Also contemplated to be useful will be antiapoptotic genes and angiogenesis promoters.

### VII. Ribozymes

Although proteins traditionally have been used for catalysis of nucleic acids, another class of macromolecules has emerged as useful in this endeavor. Ribozymes are RNA-protein complexes that cleave nucleic acids in a site-specific fashion. Ribozymes have specific catalytic domains that possess endonuclease activity (Kim and Cook, 1987; Gerlach *et al.,* 1987; Forster and Symons, 1987). For example, a large number of ribozymes accelerate phosphoester transfer reactions with a high degree of specificity, often cleaving only one of several phosphoesters in an oligonucleotide substrate (Michel and Westhof, 1990; Reinhold-Hurek and Shub, 1992). This specificity has been attributed to the requirement that the substrate bind *via* specific base-pairing interactions to the internal guide sequence ("IGS") of the ribozyme prior to chemical reaction.

Ribozyme catalysis has primarily been observed as part of sequence-specific cleavage/ligation reactions involving nucleic acids (Joyce, 1989; Cook *et al.,* 1981). For example, U.S. Patent 5,354,855 reports that certain ribozymes can act as endonucleases with a sequence specificity greater than that of known ribonucleases and approaching that of the DNA restriction enzymes. Thus, sequence-specific ribozyme-mediated inhibition of gene expression may be particularly suited to therapeutic applications (Scanlon *et al.,* 1991; Sarver *et al.,* 1990). Recently, it was reported that ribozymes elicited genetic changes in some cells lines to which they were applied; the altered genes included the oncogenes H-ras, c-fos and genes of HIV. Most of this work involved the modification of a target mRNA, based on a specific mutant codon that is cleaved by a specific ribozyme. Targets for this embodiment will include angiogenic genes such as VEGFs and angiopoeiteins as well as the oncogenes (*e.g., ras, myc, neu, raf, erb, src, fms, jun, trk, ret, hst, gsp, bcl-2, EGFR, grb2* and *abl*)*.*

### VIII. Single Chain Antibodies

In yet another embodiment, one gene may comprise a single-chain antibody. Methods for the production of single-chain antibodies are well known to those of skill in the art. The skilled artisan is referred to U.S. Patent 5,359,046, for such methods. A single chain antibody is created by fusing together the variable domains of the heavy and light chains using a short peptide linker, thereby reconstituting an antigen binding site on a single molecule.

Single-chain antibody variable fragments (scFvs) in which the C-terminus of one variable domain is tethered to the N-terminus of the other *via* a 15 to 25 amino acid peptide or linker, have been developed without significantly disrupting antigen binding or specificity of the binding (Bedzyk *et al.,* 1990; Chaudhary *et al.,* 1990): These Fvs lack the constant regions (Fc) present in the heavy and light chains of the native antibody.

Antibodies to a wide variety of molecules are contemplated, such as oncogenes, growth factors, hormones, enzymes, transcription factors or receptors. Also contemplated are secreted antibodies, targeted to serum, against angiogenic factors (VBGF/VSP; βFGF; αFGF) and endothelial antigens necessary for angiogenesis (*i.e*., V3 integrin). Specifically contemplated are growth factors such as transforming growth factor and platelet derived growth factor.

### IX. Cell Cycle Regulators

Cell cycle regulators provide possible advantages, when combined with other genes. Such cell cycle regulators include p27, p21, p57, p18, p73, p19, p15, E2F-1, E2F-2, E2F-3, p107, p130 and E2F-4. Other cell cycle regulators include anti-angiogenic proteins, such as soluble Flt1 (dominant negative soluble VEGF receptor), soluble Wnt receptors, soluble Tie2/Tek receptor, soluble hemopexin domain of matrix metalloprotease 2 and soluble receptors of other angiogenic cytokines (*e.g*. VBGFR1/KDR, VEGFR3/F1t4, both VEGF receptors).

### X. Chemokines

Genes that code for chemokines also may be used in the present invention. Chemokines generally act as chemoattractants to recruit immune effector cells to the site of chemokine expression. It may be advantageous to express a particular chemokine gene in combination with, for example, a cytokine gene, to enhance the recruitment of other immune system components to the site of treatment. Such chemokines include RANTES, MCAF, MIP1-α, MIP1-β and IP-10. The skilled artisan will recognize that certain cytokines are also known to have chemoattractant effects and could also be classified under the term chemokines.

### D. Expression Constructs

### I. Vectors

The term "vector" is used to refer to a carrier nucleic acid molecule into which a nucleic acid sequence can be inserted for introduction into a cell where it can be replicated. A nucleic acid sequence can be "exogenous," which means that it is foreign to the cell into which the vector is being introduced or that the sequence is homologous to a sequence in the cell but in a position within the host cell nucleic acid in which the sequence is ordinarily not found. Vectors include plasmids, cosmids, viruses (bacteriophage, animal viruses, and plant viruses), and artificial chromosomes (*e.g.,* YACs). One of skill in the art would be well equipped to construct a vector through standard recombinant techniques (see, for example, Goodbourn and Maniatis *et al*., 1988 and Ausubel *et al.,* 1994, both incorporated herein by reference).

The term "expression vector" refers to any type of genetic construct comprising a nucleic acid coding for a RNA capable of being transcribed. In some cases, RNA molecules are then translated into a protein, polypeptide, or peptide. In other cases, these sequences are not translated, for example, in the production of antisense molecules or ribozymes. Expression vectors can contain a variety of "control sequences," which refer to nucleic acid sequences necessary for the transcription and possibly translation of an operably linked coding sequence in a particular host cell. According to the present invention, the vectors will contain sufficient portions of the Egr-1 promoter to confer chemical inducibility. In addition to control sequences that govern transcription and translation, vectors and expression vectors may contain nucleic acid sequences that serve other functions as well and are described *infra.*

### i. Initiation Signals and Internal Ribosome Binding Sites

A specific initiation signal also may be required for efficient translation of coding sequences. These signals include the ATG initiation codon or adjacent sequences. Exogenous translational control signals, including the ATG initiation codon, may need to be provided. One of ordinary skill in the art would readily be capable of determining this and providing the necessary signals. It is well known that the initiation codon must be "in-frame" with the reading frame of the desired coding sequence to ensure translation of the entire insert. The exogenous translational control signals and initiation codons can be either natural or synthetic. The efficiency of expression may be enhanced by the inclusion of appropriate transcription enhancer elements.

In certain embodiments of the invention, the use of internal ribosome entry sites (IRES) elements are used to create multigene, or polycistronic, messages. IRES elements are able to bypass the ribosome scanning model of 5' methylated Cap dependent translation and begin translation at internal sites (Pelletier and Sonenberg, 1988). IRES elements from two members of the picornavirus family (polio and encephalomyocarditis) have been described (Pelletier and Sonenberg, 1988), as well an IRES from a mammalian message (Macejak and Sarnow, 1991). IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message (see U.S. Patent Nos. 5,925,565 and 5,935,819, ).

### ii. Multiple Cloning Sites

Vectors can include a multiple cloning site (MCS), which is a nucleic acid region that contains multiple restriction enzyme sites, any of which can be used in conjunction with standard recombinant technology to digest the vector (see, for example, Carbonelli *et al.,* 1999, Levenson *et al.,* 1998, and Cocea, 1997,) "Restriction enzyme digestion" refers to catalytic cleavage of a nucleic acid molecule with an enzyme that functions only at specific locations in a nucleic acid molecule. Many of these restriction enzymes are commercially available. Use of such enzymes is widely understood by those of skill in the art. Frequently, a vector is linearized or fragmented using a restriction enzyme that cuts within the MCS to enable exogenous sequences to be ligated to the vector. "Ligation" refers to the process of forming phosphodiester bonds between two nucleic acid fragments, which may or may not be contiguous with each other. Techniques involving restriction enzymes and ligation reactions are well known to those of skill in the art of recombinant technology.

### iii. Splicing Sites

Most transcribed eukaryotic RNA molecules will undergo RNA splicing to remove introns from the primary transcripts. Vectors containing genomic eukaryotic sequences may require donor and/or acceptor splicing sites to ensure proper processing of the transcript for protein expression (see, for example, Chandler *et al.,* 1997)

### iv. Termination Signals

The vectors or constructs of the present invention will generally comprise at least one termination signal. A "termination signal" or "terminator" is comprised of the DNA sequences involved in specific termination of an RNA transcript by an RNA polymerase. Thus, in certain embodiments a termination signal that ends the production of an RNA transcript is contemplated. A terminator may be necessary *in vivo* to achieve desirable message levels.

In eukaryotic systems, the terminator region may also comprise specific DNA sequences that permit site-specific cleavage of the new transcript so as to expose a polyadenylation site. This signals a specialized endogenous polymerase to add a stretch of about 200 A residues (polyA) to the 3' end of the transcript. RNA molecules modified with this polyA tail appear to more stable and are translated more efficiently. Thus, in other embodiments involving eukaryotes, it is preferred that that terminator comprises a signal for the cleavage of the RNA, and it is more preferred that the terminator signal promotes polyadenylation of the message. The terminator and/or polyadenylation site elements can serve to enhance message levels and to minimize read through from the cassette into other sequences.

Terminators contemplated for use in the invention include any known terminator of transcription described herein or known to one of ordinary skill in the art, including but not limited to, for example, the termination sequences of genes, such as for example the bovine growth hormone terminator or viral termination sequences, such as for example the SV40 terminator. In certain embodiments, the termination signal may be a lack of transcribable or translatable sequence, such as due to a sequence truncation.

### v. Polyadenylation Signals

In expression, particularly eukaryotic expression, one will typically include a polyadenylation signal to effect proper polyadenylation of the transcript. The nature of the polyadenylation signal is not believed to be crucial to the successful practice of the invention, and any such sequence may be employed. Preferred embodiments include the SV40 polyadenylation signal or the bovine growth hormone polyadenylation signal, convenient and known to function well in various target cells. Polyadenylation may increase the stability of the transcript or may facilitate cytoplasmic transport.

### vi. Origins of Replication

In order to propagate a vector in a host cell, it may contain one or more origins of replication sites (often termed "ori"), which is a specific nucleic acid sequence at which replication is initiated. Alternatively an autonomously replicating sequence (ARS) can be employed if the host cell is yeast.

### vii. Selectable and Screenable Markers

In certain embodiments of the invention, cells containing a nucleic acid construct of the present invention may be identified *in vitro* or *in vivo* by including a marker in the expression vector. Such markers would confer an identifiable change to the cell permitting easy identification of cells containing the expression vector. Generally, a selectable marker is one that confers a property that allows for selection. A positive selectable marker is one in which the presence of the marker allows for its selection, while a negative selectable marker is one in which its presence prevents its selection. An example of a positive selectable marker is a drug resistance marker.

Usually the inclusion of a drug selection marker aids in the cloning and identification of transformants, for example, genes that confer resistance to neomycin, puromycin, hygromycin, DHFR, GPT, zeocin and histidinol are useful selectable markers. In addition to markers conferring a phenotype that allows for the discrimination of transformants based on the implementation of conditions, other types of markers including screenable markers such as GFP, whose basis is colorimetric analysis, are also contemplated. Alternatively, screenable enzymes such as herpes simplex virus thymidine kinase (tk) or chloramphenicol acetyltransferase (CAT) may be utilized. One of skill in the art would also know how to employ immunologic markers, possibly in conjunction with FACS analysis. The marker used is not believed to be important, so long as it is capable of being expressed simultaneously with the nucleic acid encoding a gene product. Further examples of selectable and screenable markers are well known to one of skill in the art.

### viii. Plasmid Vectors

In certain embodiments, a plasmid vector is contemplated for use to transform a host cell. In general, plasmid vectors containing replicon and control sequences which are derived from species compatible with the host cell are used in connection with these hosts. The vector ordinarily carries a replication site, as well as marking sequences which are capable of providing phenotypic selection in transformed cells. In a non-limiting example, E. coli is often transformed using derivatives of pBR322, a plasmid derived from an E. coli species. pBR322 contains genes for ampicillin and tetracycline resistance and thus provides easy means for identifying transformed cells. The pBR plasmid, or other microbial plasmid or phage must also contain, or be modified to contain, for example, promoters which can be used by the microbial organism for expression of its own proteins.

In addition, phage vectors containing replicon and control sequences that are compatible with the host microorganism can be used as transforming vectors in connection with these hosts. For example, the phage lambda GEM^{™}-11 may be utilized in making a recombinant phage vector which can be used to transform host cells, such as, for example, *E*. *coli* LE392.

Further useful plasmid vectors include pIN vectors (Inouye *et al.,* 1985); and pGEX vectors, for use in generating glutathione S-transferase (GST) soluble fusion proteins for later purification and separation or cleavage. Other suitable fusion proteins are those with β-galactosidase, ubiquitin, and the like.

Bacterial host cells, for example, *E*. *coli,* comprising the expression vector, are grown in any of a number of suitable media, for example, LB. The expression of the recombinant protein in certain vectors may be induced, as would be understood by those of skill in the art, by contacting a host cell with an agent specific for certain promoters, *e.g*., by adding IPTG to the media or by switching incubation to a higher temperature. After culturing the bacteria for a further period, generally of between 2 and 24 h, the cells are collected by centrifugation and washed to remove residual media.

### ix. Viral Vectors

The ability of certain viruses to infect cells or enter cells *via* receptor-mediated endocytosis, and to integrate into host cell genome and express viral genes stably and efficiently have made them attractive candidates for the transfer of foreign nucleic acids into cells (*e.g*., mammalian cells). Non-limiting examples of virus vectors that may be used to deliver a nucleic acid of the present invention are described below.

### a. Adenoviral Vectors

A particular method for delivery of the nucleic acid involves the use of an adenovirus expression vector. Although adenovirus vectors are known to have a low capacity for integration into genomic DNA, this feature is counterbalanced by the high efficiency of gene transfer afforded by these vectors. "Adenovirus expression vector" is meant to include those constructs containing adenovirus sequences sufficient to (a) support packaging of the construct and (b) to ultimately express a tissue or cell-specific construct that has been cloned therein. Knowledge of the genetic organization or adenovirus, a 36 kb, linear, double-stranded DNA virus, allows substitution of large pieces of adenoviral DNA with foreign sequences up to 7 kb (Grunhaus and Horwitz, 1992).

### b. AAV Vectors

The nucleic acid may be introduced into the cell using adenovirus assisted transfection. Increased transfection efficiencies have been reported in cell systems using adenovirus coupled systems (Kelleher and Vos, 1994; Cotten *et al.,* 1992; Curiel, 1994). Adeno-associated virus (AAV) is an attractive vector system for use according to the present invention as it has a high frequency of integration and it can infect nondividing cells, thus making it useful for delivery of genes into mammalian cells, for example, in tissue culture (Muzyczka, 1992) or *in vivo.* AAV has a broad host range for infectivity (Tratschin *et al.,* 1984; Laughlin *et al.,* 1986; Lebkowski *et al*., 1988; McLaughlin *et al.,* 1988). Details concerning the generation and use of rAAV vectors are described in U.S. Patents 5,139,941 and 4,797,368.

### c. Retroviral Vectors

Retroviruses have promise as gene delivery vectors due to their ability to integrate their genes into the host genome, transferring a large amount of foreign genetic material, infecting a broad spectrum of species and cell types and of being packaged in special cell-lines (Miller, 1992).

In order to construct a retroviral vector, a nucleic acid is inserted into the viral genome in the place of certain viral sequences to produce a virus that is replication-defective. In order to produce virions, a packaging cell line containing the gag, pol, and env genes but without the LTR and packaging components is constructed (Mann *et al.,* 1983). When a recombinant plasmid containing a cDNA, together with the retroviral LTR and packaging sequences is introduced into a special cell line (*e.g*., by calcium phosphate precipitation for example), the packaging sequence allows the RNA transcript of the recombinant plasmid to be packaged into viral particles, which are then secreted into the culture media (Nicolas and Rubenstein, 1988; Temin, 1986; Mann *et al.,* 1983). The media containing the recombinant retroviruses is then collected, optionally concentrated, and used for gene transfer. Retroviral vectors are able to infect a broad variety of cell types. However, integration and stable expression require the division of host cells (Paskind *et al.,* 1975).

Lentiviruses are complex retroviruses, which, in addition to the common retroviral genes *gag, pol,* and *env*, contain other genes with regulatory or structural function. Lentiviral vectors are well known in the art (see, for example, Naldini *et al.,* 1996; Zufferey *et al.,* 1997; Blomer *et al*., 1997; U.S. Patents 6,013,516 and 5,994,136). Some examples of lentivirus include the Human Immunodeficiency Viruses: HIV-1, HIV-2 and the Simian Immunodeficiency Virus: SIV. Lentiviral vectors have been generated by multiply attenuating the HIV virulence genes, for example, the genes *env, vif, vpr, vpu* and *nef* are deleted making the vector biologically safe.

Recombinant lentiviral vectors are capable of infecting non-dividing cells and can be used for both *in vivo* and *ex vivo* gene transfer and expression of nucleic acid sequences. For example, recombinant lentivirus capable of infecting a non-dividing cell wherein a suitable host cell is transfected with two or more vectors carrying the packaging functions, namely gag, pol and env, as well as rev and tat is described in U.S. Patent No. 5,994,136, . One may target the recombinant virus by linkage of the envelope protein with an antibody or a particular ligand for targeting to a receptor of a particular cell-type. By inserting a sequence (including a regulatory region) of interest into the viral vector, along with another gene which encodes the ligand for a receptor on a specific target cell, for example, the vector is now target-specific.

### d. Other Viral Vectors

Other viral vectors may be employed as vaccine constructs in the present invention. Vectors derived from viruses such as vaccinia virus (Ridgeway, 1988; Baichwal and Sugden, 1986; Coupar *et al.,* 1988), sindbis virus, cytomegalovirus and herpes simplex virus may be employed. They offer several attractive features for various mammalian cells (Friedmann, 1989; Ridgeway, 1988; Baichwal and Sugden, 1986; Coupar *et al.,* 1988; Horwich *et al.,* 1990).

### e. Delivery Using Modified Viruses

A nucleic acid to be delivered may be housed within an infective virus that has been engineered to express a specific binding ligand. The virus particle will thus bind specifically to the cognate receptors of the target cell and deliver the contents to the cell. A novel approach designed to allow specific targeting of retrovirus vectors was developed based on the chemical modification of a retrovirus by the chemical addition of lactose residues to the viral envelope. This modification can permit the specific infection of hepatocytes *via* sialoglycoprotein receptors.

Another approach to targeting of recombinant retroviruses was designed in which biotinylated antibodies against a retroviral envelope protein and against a specific cell receptor were used. The antibodies were coupled *via* the biotin components by using streptavidin (Roux *et al.,* 1989). Using antibodies against major histocompatibility complex class I and class II antigens, they demonstrated the infection of a variety of human cells that bore those surface antigens with an ecotropic virus *in vitro* (Roux *et al.,* 1989).

### II. Vector Delivery and Cell Transformation

Suitable methods for nucleic acid delivery for transformation of an organelle, a cell, a tissue or an organism for use with the current invention are believed to include virtually any method by which a nucleic acid (*e.g*., DNA) can be introduced into an organelle, a cell, a tissue or an organism, as described herein or as would be known to one of ordinary skill in the art. Such methods include, but are not limited to, direct delivery of DNA such as by ex vivo transfection (Wilson *et al.,* 1989, Nabel *et al,* 1989), by injection (U.S. Patents 5,994,624, 5,981,274, 5,945,100, 5,780,448, 5,736,524, 5,702,932, 5,656,610, 5,589,466 and 5,580,859, ), including microinjection (Harlan and Weintraub, 1985; U.S. Patent No. 5,789,215,; by electroporation (U.S. Patent No. 5,384,253, Tur-Kaspa *et al.,* 1986; Potter *et al.,* 1984); by calcium phosphate precipitation (Graham and Van Der Eb, 1973; Chen and Okayama, 1987; Rippe *et al.,* 1990); by using DEAE-dextran followed by polyethylene glycol (Gopal, 1985); by direct sonic loading (Fechheimer *et al.,* 1987); by liposome mediated transfection (Nicolau and Sene, 1982; Fraley *et al.,* 1979; Nicolau *et al.,* 1987; Wong *et al.,* 1980; Kaneda *et al.,* 1989; Kato *et al.,* 1991) and receptor-mediated transfection (Wu and Wu, 1987; Wu and Wu, 1988); by microprojectile bombardment (PCT Application Nos. WO 94/09699 and 95/06128; U.S. Patents 5,610,042; 5,322,783 5,563,055, 5,550,318, 5,538,877 and 5,538,880 ); by agitation with silicon carbide fibers (Kaeppler et al., 1990; U.S. Patents 5,302,523 and 5,464,765); by *Agrobacterium*-mediated transformation (U.S. Patents 5,591,616 and 5,563,055); by PEG-mediated transformation of protoplasts (Omirulleh et al., 1993; U.S. Patents 4,684,611 and 4,952,500); by desiccation/inhibition-mediated DNA uptake (Potrykus *et al.,* 1985), and any combination of such methods. Through the application of techniques such as these, organelle(s), cell(s), tissue(s) or organism(s) may be stably or transiently transformed.

### i. Ex Vivo Transformation

Methods for tranfecting vascular cells and tissues removed from an organism in an *ex vivo* setting are known to those of skill in the art. For example, cannine endothelial cells have been genetically altered by retrovial gene tranfer in vitro and transplanted into a canine (Wilson *et al.,* 1989). In another example, yucatan minipig endothelial cells were tranfected by retrovirus in vitro and transplated into an artery using a double-ballonw catheter (Nabel *et al.,* 1989). Thus, it is contemplated that cells or tissues may be removed and tranfected ex vivo using the nucleic acids of the present invention. In particular aspects, the transplanted cells or tissues may be placed into an organism. In preferred facets, a nucleic acid is expressed in the transplated cells or tissues.

### ii. Injection

In certain embodiments, a nucleic acid may be delivered to an organelle, a cell, a tissue or an organism via one or more injections (*i.e*., a needle injection), such as, for example, subcutaneously, intradermally, intramuscularly, intervenously, intraperitoneally, etc. Methods of injection of vaccines are well known to those of ordinary skill in the art (*e.g*., injection of a composition comprising a saline solution). Further embodiments of the present invention include the introduction of a nucleic acid by direct microinjection. Direct microinjection has been used to introduce nucleic acid constructs into *Xenopus* oocytes (Harland and Weintraub, 1985).

### iii. Electroporation

In certain embodiments of the present invention, a nucleic acid is introduced into an organelle, a cell, a tissue or an organism *via* electroporation. Electroporation involves the exposure of a suspension of cells and DNA to a high-voltage electric discharge. In some variants of this method, certain cell wall-degrading enzymes, such as pectin-degrading enzymes, are employed to render the target recipient cells more susceptible to transformation by electroporation than untreated cells (U.S. Patent No. 5,384,253 ). Alternatively, recipient cells can be made more susceptible to transformation by mechanical wounding.

Transfection of eukaryotic cells using electroporation has been quite successful. Mouse pre-B lymphocytes have been transfected with human kappa-immunoglobulin genes (Potter *et al.,* 1984), and rat hepatocytes have been transfected with the chloramphenicol acetyltransferase gene (Tur-Kaspa *et al.,* 1986) in this manner.

### iv. Calcium Phosphate

In other embodiments of the present invention, a nucleic acid is introduced to the cells using calcium phosphate precipitation. Human KB cells have been transfected with adenovirus 5 DNA (Graham and Van Der Eb, 1973) using this technique. Also in this manner, mouse L(A9), mouse C127, CHO, CV-1, BHK, NIH3T3 and HeLa cells were transfected with a neomycin marker gene (Chen and Okayama, 1987), and rat hepatocytes were transfected with a variety of marker genes (Rippe *et al.,* 1990).

### v. DEAE-Dextran

In another embodiment, a nucleic acid is delivered into a cell using DEAE-dextran followed by polyethylene glycol. In this manner, reporter plasmids were introduced into mouse myeloma and erythroleukemia cells (Gopal, 1985).

### vi. Sonication Loading

Additional embodiments of the present invention include the introduction of a nucleic acid by direct sonic loading. LTK- fibroblasts have been transfected with the thymidine kinase gene by sonication loading (Fechheimer *et al.,* 1987).

### vii. Liposome-Mediated Transfection

In a further embodiment of the invention, a nucleic acid may be entrapped in a lipid complex such as, for example, a liposome. Liposomes are vesicular structures characterized by a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh and Bachhawat, 1991). Also contemplated is an nucleic acid complexed with Lipofectamine (Gibco BRL) or Superfect (Qiagen).

Liposome-mediated nucleic acid delivery and expression of foreign DNA *in vitro* has been very successful (Nicolau and Sene, 1982; Fraley *et al*., 1979; Nicolau *et al*., 1987). The feasibility of liposome-mediated delivery and expression of foreign DNA in cultured chick embryo, HeLa and hepatoma cells has also been demonstrated (Wong *et al*., 1980).

In certain embodiments of the invention, a liposome may be complexed with a hemagglutinating virus (HVJ). This has been shown to facilitate fusion with the cell membrane and promote cell entry of liposome-encapsulated DNA (Kaneda *et al.,* 1989). In other embodiments, a liposome may be complexed or employed in conjunction with nuclear non-histone chromosomal proteins (HMG-1) (Kato *et al.,* 1991). In yet further embodiments, a liposome may be complexed or employed in conjunction with both HVJ and HMG-1. In other embodiments, a delivery vehicle may comprise a ligand and a liposome.

### viii. Receptor Mediated Transfection

Still further, a nucleic acid may be delivered to a target cell via receptor-mediated delivery vehicles. These take advantage of the selective uptake of macromolecules by receptor-mediated endocytosis that will be occurring in a target cell. In view of the cell type-specific distribution of various receptors, this delivery method adds another degree of specificity to the present invention.

Certain receptor-mediated gene targeting vehicles comprise a cell receptor-specific ligand and a nucleic acid-binding agent. Others comprise a cell receptor-specific ligand to which the nucleic acid to be delivered has been operatively attached. Several ligands have been used for receptor-mediated gene transfer (Wu and Wu, 1987; Wagner *et al.,* 1990; Perales *et al.,* 1994; Myers, EPO 0273085), which establishes the operability of the technique. Specific delivery in the context of another mammalian cell type has been described (Wu and Wu, 1993 ). In certain aspects of the present invention, a ligand will be chosen to correspond to a receptor specifically expressed on the target cell population.

In other embodiments, a nucleic acid delivery vehicle component of a cell-specific nucleic acid targeting vehicle may comprise a specific binding ligand in combination with a liposome. The nucleic acid(s) to be delivered are housed within the liposome and the specific binding ligand is functionally incorporated into the liposome membrane. The liposome will thus specifically bind to the receptor(s) of a target cell and deliver the contents to a cell. Such systems have been shown to be functional using systems in which, for example, epidermal growth factor (EGF) is used in the receptor-mediated delivery of a nucleic acid to cells that exhibit upregulation of the EGF receptor.

In still further embodiments, the nucleic acid delivery vehicle component of a targeted delivery vehicle may be a liposome itself, which will preferably comprise one or more lipids or glycoproteins that direct cell-specific binding. For example, lactosyl-ceramide, a galactose-terminal asialganglioside, have been incorporated into liposomes and observed an increase in the uptake of the insulin gene by hepatocytes (Nicolau *et al.,* 1987). It is contemplated that the tissue-specific transforming constructs of the present invention can be specifically delivered into a target cell in a similar manner.

### E. Combined Administration of Therapeutic Genes and DNA Damaging Agents

### I. Adminstration

Tumors that can be treated with the present invention include, but are not limited to, tumors of the brain (glioblastomas, medulloblastoma, astrocytoma, oligodendroglioma, ependymomas), lung, liver, spleen, kidney, lymph node, small intestine, pancreas, blood cells, colon, stomach, breast, endometrium, prostate, testicle, ovary, skin, head and neck, esophagus, bone marrow, blood or other tissue. The tumor may be distinguished as metastatic and non-metastatic. Various embodiments include tumor cells of the skin, muscle, facia, brain, prostate, breast, endometrium, lung, head & neck, pancreas, small intestine, blood cells, liver, testes, ovaries, colon, skin, stomach, esophagus, spleen, lymph node, bone marrow or kidney. Other embodiments include fluid samples such as peripheral blood, lymph fluid, ascites, serous fluid, pleural effusion, sputum, cerebrospinal fluid, lacrimal fluid, stool or urine.

In accordance with the present invention, delivery of an Egr-1-driven expression vector and a DNA damaging agent is provided. This combination is capable of affecting a hyperproliferative disease (*e.g*., cancer) in a subject, for example, by killing one or more target cells, inducing apoptosis in one or more target cells, reducing the growth rate of one or more target cells, reducing the incidence or number of metastases, reducing a tumor's size, inhibiting a tumor's growth, reducing the blood supply to a tumor or one or more target cells, promoting an immune response against one or more target cells or a tumor, preventing or inhibiting the progression of a cancer, or increasing the lifespan of a subject with a cancer.

More generally, the agents are provided in a combined amount with an effective dose to kill or inhibit proliferation of a cancer cell. This process may involve contacting the cell(s) with the agents at the same time or within a period of time wherein separate administration of the agents to a cell, tissue or organism produces a desired therapeutic benefit. This may be achieved by contacting the cell, tissue or organism with a single composition or pharmacological formulation that includes both agents, or by contacting the cell with two or more distinct compositions or formulations.

The terms "contacted" and "exposed," when applied to a cell, tissue or organism, are used herein to describe the process by which a therapeutic construct and DNA damaging agent are delivered to a target cell, tissue or organism or are placed in direct juxtaposition with the target cell, tissue or organism. To achieve cell killing or stasis, the agents are delivered to one or more cells in a combined amount effective to kill the cells or prevent them from dividing.

The expression construct may precede, be concurrent with and/or follow the DNA-damaging agent by intervals ranging from minutes to weeks. In embodiments where the agents are applied separately to a cell, tissue or organism, one would generally ensure that a significant period of time did not expire between the time of each delivery, such that the DNA damaging agent would still be able to induce expression from the the Egr-1 promoter in the cell, tissue or organism. For example, in such instances, it is contemplated that one may contact the cell, tissue or organism with the agents substantially simultaneously (*i.e*., within less than about a minute). In other aspects, the agents may be administered about 1 minute, about 5 minutes, about 10 minutes, about 20 minutes about 30 minutes, about 45 minutes, about 60 minutes, about 2 hours, about 3 hours, about 4 hours, about 5 hours, about 6 hours, about 7 hours about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 22 hours, about 23 hours, about 24 hours, about 25 hours, about 26 hours, about 27 hours, about 28 hours, about 29 hours, about 30 hours, about 31 hours, about 32 hours, about 33 hours, about 34 hours, about 35 hours, about 36 hours, about 37 hours, about 38 hours, about 39 hours, about 40 hours, about 41 hours, about 42 hours, about 43 hours, about 44 hours, about 45 hours, about 46 hours, about 47 hours, about 48 hours, about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 21 days, about 1, about 2, about 3, about 4, about 5, about 6, about 7 or about 8 weeks or more apart, and any range derivable therein.

Various combination may be employed. Non-limiting examples of such combinations are shown below, wherein an Egr-1 vector is "A" and a DNA damaging agent is "B":

| | | | | | | | |
|---|---|---|---|---|---|---|---|
| A/B/A | B/A/B | B/B/A | A/A/B | A/B/B | B/A/A | A/B/B/B | B/A/B/B |
| B/B/B/A | B/B/A/B | | A/A/B/B | A/B/A/B | | A/B/B/A | B/B/A/A |
| B/A/B/A | B/A/A/B | | A/A/A/B | B/A/A/A | | A/B/A/A | A/A/B/A |

Other combinations are also contemplated.

Administration of the agents of the present invention may follow general protocols for the administration of chemo- or gene therapeutics, taking into account the toxicity, if any. It is expected that the treatment cycles would be repeated as necessary. In particular embodiments, it is contemplated that various additional agents may be applied in any combination with the present invention. ,

"Effective amount" is defined as an amount of the agent that will decrease, reduce, inhibit or otherwise abrogate the growth of a cancer cell, induce apoptosis, inhibit metastasis, kill cells or induce cytotoxicity in cells.

The agents may, in general, be administered intravenously, intraarterially, intratumorally, parenterally or intraperitoneally. In particular, it is envisioned that local, regional and systemic delivery of the Egr-1 vector and DNA damaging agents to patients with cancers all will be suitable methods. A local administration also is useful, and includes direct injection of tumor mass, circumferential injection, and injections or bathing of a resected tumor bed. Regional delivery may include administration into the tumor vasculature or regional blood supply. Alternatively, systemic delivery of either or both DNA damaging agents and Egr-1 vector is appropriate in certain circumstances, for example, where extensive metastasis has occurred.

### II. Formulations

The pharmaceutical forms of the agents are generally prepared for use as injectable solutions or dispersions. In all cases, the form should be sterile and must be fluid to the extent that easy syringability exists. It also should be stable under the conditions of manufacture and storage, and be preserved against the contaminating action of microorganisms, such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

As used herein, "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

### F. Adjunct Therapies

### I. Radiotherapeutic Agents

Radiotherapeutic agents and factors include radiation and waves that induce DNA damage for example, γ-irradiation, X-rays, UV-irradiation, microwaves, electronic emissions, radioisotopes, and the like. Therapy may be achieved by irradiating the localized tumor site with the above, described forms of radiations. It is most likely that all of these factors effect a broad range of damage DNA, on the precursors of DNA, the replication and repair of DNA, and the assembly and maintenance of chromosomes.

Dosage ranges for X-rays range from daily doses of 50 to 200 roentgens for prolonged periods of time (3 to 4 weeks), to single doses of 2000 to 6000 roentgens. Dosage ranges for radioisotopes vary widely, and depend on the half-life of the isotope, the strength and type of radiation emitted, and the uptake by the neoplastic cells.

### II. Surgery

Surgical treatment for removal of the cancerous growth is generally a standard procedure for the treatment of tumors and cancers. This attempts to remove the entire cancerous growth or to reduce it in order to make another therapy more effective, *e.g.*, combined with chemotherapy and/or radiotherapy to ensure the destruction of any remaining neoplastic or malignant cells. Thus, surgery may be used in combination with the present invention.

Approximately 60% of persons with cancer will undergo surgery of some type, which includes, for example, preventative, diagnostic or staging, curative and palliative surgery. Surgery, and in particular a curative surgery, may be used in conjunction with other therapies; such as the present invention and one or more other agents.

Curative surgery includes resection in which all or part of cancerous tissue is physically removed, excised and/or destroyed. It is further contemplated that surgery may remove, excise or destroy superficial cancers, precancers, or incidental amounts of normal tissue. Treatment by surgery includes for example, tumor resection, laser surgery, cryosurgery, electrosurgery, and miscopically controlled surgery (Mohs' surgery). Tumor resection refers to physical removal of at least part of a tumor. Upon excision of part of all of cancerous cells, tissue, or tumor, a cavity may be formed in the body.

Further treatment of the tumor or area of surgery may be accomplished treatment of the patient or surgical field with an additional anti-cancer therapy. Such treatment may be repeated, for example, about every 1, about every 2, about every 3, about every 4, about every 5, about every 6, or about every 7 days, or about every 1, about every 2, about every 3, about every 4, or about every 5 weeks or about every 1, about every 2, about every 3, about every 4, about every 5, about every 6, about every 7, about every 8, about every 9, about every 10, about every 11, or about every 12 months. These treatments may be of varying dosages as well.

### III. Immune Therapy

An immunotherapeutic agent generally relies on the use of immune effector cells and molecules to target and destroy cancer cells. The immune effector may be, for example, an antibody specific for some marker on the surface of a tumor cell. The antibody alone may serve as an effector of therapy or it may recruit other cells to actually effect cell killing. The antibody also may be conjugated to a drug or toxin (*e.g*., a chemotherapeutic, a radionuclide, a ricin A chain, a cholera toxin, a pertussis toxin, *etc.*) and serve merely as a targeting agent. Such antibody conjugates are called immunotoxins, and are well known in the art (see U.S. Patent 5,686,072, U.S. Patent 5,578,706, U.S. Patent 4,792,447, U.S. Patent 5,045,451, U.S. Patent 4,664,911, and U.S. Patent 5,767,072). Alternatively, the effector, may be a lymphocyte carrying a surface molecule that interacts, either directly or indirectly, with a tumor cell target. Various effector cells include cytotoxic T cells and NK cells.

In one aspect of immunotherapy, the tumor cell must bear some marker that is amenable to targeting, *i.e.*, is not present on the majority of other cells. Many tumor markers exist and any of these may be suitable for targeting in the context of the present invention. Common tumor markers include carcinoembryonic antigen, prostate specific antigen, urinary tumor associated antigen, fetal antigen, tyrosinase (p97), gp68, TAG-72, HMFG, Sialyl Lewis Antigen, MucA, MucB, PLAP, estrogen receptor, laminin receptor, *erb* B and p155.

### i. Immune Stimulators

In a specific aspect of immunotherapy is to use an immune stimulating molecule as an agent, or more preferably in conjunction with another agent, such as for example, a cytokines such as for example IL-2, IL-4, IL-12, GM-CSF, tumor necrosis factor; interferons alpha, beta, and gamma; F42K and other cytokine analogs; a chemokine such as for example MIP-1, MIP-1β, MCP-1, RANTES, IL-8; or a growth factor such as for example FLT3 ligand.

One particular cytokine contemplated for use in the present invention is tumor necrosis factor. Tumor necrosis factor (TNF; Cachectin) is a glycoprotein that kills some kinds of cancer cells, activates cytokine production, activates macrophages and endothelial cells, promotes the production of collagen and collagenases, is an inflammatory mediator and also a mediator of septic shock, and promotes catabolism, fever and sleep. Some infectious agents cause tumor regression through the stimulation of TNF production. TNF can be quite toxic when used alone in effective doses, so that the optimal regimens probably will use it in lower doses in combination with other drugs. Its immunosuppressive actions are potentiated by gammainterferon, so that the combination potentially is dangerous. A hybrid of TNF and interferon-α also has been found to possess anti-cancer activity.

Another cytokine specifically contemplate is interferon alpha. Interferon alpha has been used in treatment of hairy cell leukemia, Kaposi's sarcoma, melanoma, carcinoid, renal cell cancer, ovary cancer, bladder cancer, non-Hodgkin's lymphomas, mycosis fungoides, multiple myeloma, and chronic granulocytic leukemia.

### ii. Passive Immunotherapy

A number of different approaches for passive immunotherapy of cancer exist. They may be broadly categorized into the following: injection of antibodies alone; injection of antibodies coupled to toxins or chemotherapeutic agents; injection of antibodies coupled to radioactive isotopes; injection of anti-idiotype antibodies; and finally, purging of tumor cells in bone marrow.

Preferably, human monoclonal antibodies are employed in passive immunotherapy, as they produce few or no side effects in the patient. However, their application is somewhat limited by their scarcity and have so far only been administered intralesionally. For example, human monoclonal antibodies to ganglioside antigens have been administered intralesionally to patients suffering from cutaneous recurrent melanoma (Irie & Morton, 1986). Regression was observed in six out of ten patients, following, daily or weekly, intralesional injections. In another study, moderate success was achieved from intralesional injections of two human monoclonal antibodies (Irie *et al*., 1989).

It may be favorable to administer more than one monoclonal antibody directed against two different antigens or even antibodies with multiple antigen specificity. Treatment protocols also may include administration of lymphokines or other immune enhancers (Bajorin *et al.* 1988).

### iii. Active Immunotherapy

In active immunotherapy, an antigenic peptide, polypeptide or protein, or an autologous or allogenic tumor cell composition or "vaccine" is administered, generally with a distinct bacterial adjuvant (Ravindranath & Morton, 1991; Morton & Ravindranath, 1996; Morton *et al.,* 1992; Mitchell *et al.,* 1990; Mitchell *et al.,* 1993). In melanoma immunotherapy, those patients who elicit high IgM response often survive better than those who elicit no or low IgM antibodies (Morton *et al.,* 1992). IgM antibodies are often transient antibodies and the exception to the rule appears to be anti-ganglioside or anticarbohydrate antibodies.

### iv. Adoptive Immunotherapy

In adoptive immunotherapy, the patient's circulating lymphocytes, or tumor infiltrated lymphocytes, are isolated *in vitro,* activated by lymphokines such as IL-2 or transduced with genres for tumor necrosis, and readministered (Rosenberg *et al.,* 1988; 1989). To achieve this, one would administer to an animal, or human patient, an immunologically effective amount of activated lymphocytes in combination with an adjuvant-incorporated anigenic peptide composition as described herein. The activated lymphocytes will most preferably be the patient's own cells that were earlier isolated from a blood or tumor sample and activated (or "expanded") *in vitro.* This form of immunotherapy has produced several cases of regression of melanoma and renal carcinoma, but the percentage of responders were few compared to those who did not respond.

### G. Screening and Monitoring Effectiveness of Therapy

It is contemplated that in the context of the present invention one may remove cells, either tumor, normal or both tumor and normal cells, from an individual in order to either monitor the progress of treatment or as a part of the treatment. It is expected that one may monitor the effectiveness of treatment by removing such cells and treating such cells with DAPI staining to determine the level of chromatin condensation, measuring the level of apoptosis, measuring the level of neutral sphingomyelinase production or other methods such as the following.

One particular method for determining induction of apoptosis is terminal deoxynucleotidyl transferase mediated dUTP-biotin nick end labeling (TUNEL) assays, which measure the integrity of DNA (Gorczyca, 1993). This assay measures the fragmentation of DNA by monitoring the incorporation of labeled UTP into broken DNA strands by the enzyme terminal transferase. The incorporation can be monitored by electroscopy or by cell sorting methodologies (*e.g.*, FACS).

### H. Ex vivo Delivery

In the Present invention, it is contemplated that *ex vivo* gene therapy - isolation of cells from an animal or patient, treatment of the cells *in vitro,* and then the return of the modified cells back into an animal or individual - may be employed. This approach permits higher doses of therapy, and the addition of other factors that are may not be possible in an *in vivo* setting. In particular, autologous bone marrow cell (BMC) transplantation is used as a salvage procedure in which blood or bone marrow is taken and stored prior to an intensification of radiation or chemotherapy. Treatment of such cells to prevent reintroduction of cancer cells is highly beneficial.

In preparing human mononuclear cells (MNC), an aliquot of marrow is layered into a receptacle such as a centrifuge tube. Initially, MNC may be obtained from a source of bone marrow, *e.g.*, tibiae, femora, spine, ribs, hips, sternum, as well as the humeri, radi, ulna, tibiae, and fibulae. Additionally, these cells also can be obtained from cord blood, peripheral blood, or cytokine-mobilized peripheral blood. Other sources of human hematopoietic stem cells include embryonic yolk sac, fetal liver, fetal and adult spleen, and blood. The marrow layer is centrifuge to produce a pellet of red cells at the bottom of the tube, a clear layer of media, an interface layer which contains the MNC and a plasma medium layer on top. The interface layer may then be removed using, for example, suction. Centrifugation of this layer at 1000g ultimately yields a MNC pellet. This pellet may then be resuspended in a suitable buffer for cell sorting by FAGS. The isolated MNC are cloned *in vitro* to expand the of immunologically active cells. The expanded, therapeutically active cells are then provided to the patient to obtain at therapeutic effect

### I. Clinical Trials

This example is concerned with the development of human treatment protocols by methods comprising: a) providing an expression construct comprising a nucleic acid segment encoding a cancer therapeutic protein, the nucleic acid segment being positioned under the control of an Egr-1 promoter; and b) administering the expression construct to a human subject in combination with a DNA damaging compound that can induce free radicals. The methods may further comprise administering to the patient other cancer therapeutic compounds, ionizing radiation and/or any other adjunct cancer therapy. These methods will be of use in the clinical treatment of various cancers/tumors and diseases in which transformed or cancerous cells play a role. Such treatment will be particularly useful tools in anti-tumor therapy, for example, in treating patients with lung cancer, prostate cancer, ovarian cancer, testicular cancer, brain cancer, skin cancer, colon cancer, gastric cancer, esophageal cancer, tracheal cancer, head & neck cancer, pancreatic cancer, liver cancer, breast cancer, ovarian cancer, lymphoid cancer, leukemia, cervical cancer, or vulvar cancer.

The free radical-inducing DNA damaging compound may be a platinum compound such as cisplatin, a nitrogen mustard, cytoxan, cyclophosphamide, mitomycin c, adriamycin, iphosphamide, bleomycin, doxourbicin, procarbazine, actinomycin, chlorambucil, carboplatinum, busulfan, bcnu, ccnu, hexamethylmelamineoxaliplatin, epirubicin, daunorubicin, camptothecin, or mitoxantrone. Any protein with anti-cancer properties may be used and examples of such compounds are described elsewhere in the specification.

The various elements of conducting a clinical trial, including patient treatment and monitoring, are known to those of skill in the art in light of the present disclosure. The following information is being presented as a general guideline for use in establishing clinical trials using the methods of the invention.

Candidates for the phase 1 clinical trial will be patients on which all conventional therapies have failed. The therapeutic formulations of the invention will be administered on a tentative weekly basis. Effectiveness of the therapy and disease course can be assessed by monitoring parameters such as tumor size, presence of tumor markers, and/or bone marrow infiltration of cancer cells on a periodic basis. Tests that will be used to monitor the progress of the patients and the effectiveness of the treatments include: physical exam, X-ray, blood work and other clinical laboratory methodologies. In addition, peripheral blood and bone marrow samples will be drawn to assess the expression of the anticancer protein expressed by the vector The doses given in the phase 1 study will be escalated as is done in standard phase 1 clinical phase trials, *i.e.*, doses will be escalated until maximal tolerable ranges are reached.

Clinical responses may be defined by acceptable measure. For example, a complete response may be defined by complete disappearance of evidence of cancer cells for at least 2 months. Whereas a partial response may be defined by a 50% reduction of cancer cells for at least 2 months.

The typical course of treatment will vary depending upon the individual patient and disease being treated in ways known to those of skill in the art. A typical treatment course may comprise about six doses delivered over a 7 to 21 day period. Upon election by the clinician the regimen may be continued with six doses every three weeks or on a less frequent (monthly, bimonthly, quarterly etc.) basis. For example, a patient with lung cancer might be treated in eight week cycles, although longer duration may be used if no adverse effects are observed with the patient, and shorter terms of treatment may result if the patient does not tolerate the treatment as hoped. Each cycle will consist of between 20 and 35 individual doses spaced equally, although this too may be varied depending on the clinical situation. Of course, these are only exemplary times for treatment, and the skilled practitioner will readily recognize that many other time-courses are possible.

patients may, but need not, have received previous or concurrent surgical, chemo-, radio-or gene therapeutic treatments. Optimally the patient will exhibit adequate bone marrow function (defined as peripheral absolute granulocyte count of > 2,000/mm³ and platelet count of 100, 000/mm³, adequate liver function (bilirubin 1.5mg/dl) and adequate renal function (creatinine 1.5mg/dl).

The therapeutic compositions of the present invention will typically be administered parenterally in dosage unit formulations containing standard, well known non-toxic physiologically acceptable carriers, adjuvants, and vehicles as desired. The term parenteral as used herein includes intravenous, introtumoral, subcutaneous, intramuscular, intra, or infusion techniques. These compositions will be provided in an amount effective to kill or inhibit the proliferation of the cell.

Regional delivery of the compositions are an efficient method for delivering a therapeutically effective dose to counteract the clinical disease. Alternatively, systemic delivery may be appropriate. The therapeutic compositions of the present invention may be administered to the patient directly at the site of the tumor. The volume of the composition should usually be sufficient to ensure that the entire surface of the tumor is contacted by the therapeutic composition. In one embodiment, administration simply entails injection of the therapeutic composition into the tumor. In another embodiment, a catheter is inserted into the site of the tumor and the cavity may be continuously perfused for a desired period of time.

Of course, the above-described treatment regimes may be altered in accordance with the knowledge gained from pre-clinical trials. Those of skill in the art will be able to take the information disclosed in this specification and optimize treatment regimes.

### J. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice.

### EXAMPLE 1

### Methods

**Cells and cell culture**. Cell lines Seg-1, a human esophageal adenocarcinoma (Dr. David Beer, University of Michigan, Ann Arbor, MI) and DHD/K12/TRb (PROb), a rat colon adenocarcinoma established in syngeneic BD-IX rats by 1,2-dimethylhydrazine induction (Dr. Francois Martin, University of Dijon, France) were maintained in Dulbecco's Modified Eagle Medium (DMEM) (GibcoBRL, Grand Island, NY) supplemented with Fetal Bovine Serum (FBS, 10% v/v) (Intergen, Purchase, NY), penicillin (100 IU/ml), and streptomycin (100 µg/ml) (GibcoBRL), at 37°C and 7.5% CO₂.

**Animals**. Athymic nude mice, (Frederick Cancer Research Institute, Frederick, MD) received food and water *ad libitum.* Experiments were in accordance with the guidelines of the University of Chicago.

**Viral vectors**. The viral vectors Ad.Egr.TNF.11D and Ad.Null.3511.11D (GenVec, Gaithersburg, MD), were stored at -80°C, and diluted to the appropriate concentration in formulation buffer.

***In vitro* measurement of TNF-α protein**. Seg-1 and PROb cells were plated at 10⁵ cells/well in 12-well plates (Becton Dickinson, Bedford, MA), grown overnight, and infected with either Ad.Null.3511.11D or Ad.Egr.TNF.11D at 100 multiplicities of infection (MOI) in serum-free medium for 2-3 hours. IR treated cells in complete medium were exposed to 5 Gy using a Pantak PCM 1000 x-ray generator. Cells in the cisplatin group were exposed to 5 µM cisplatin in complete medium. Cells and supernatants were harvested by scraping at 24, 48, and 72 hours, and production of human TNF-α was quantified by ELISA (R&D Systems, Minneapolis, MN) following three cycles of freeze-thaw lysis. Assays were performed in triplicate. Duplicate treatment plates were used to adjust for the cytotoxicity of IR and cisplatin. Cells were harvested using versene (0.02% EDTA in HBSS) and trypsin-EDTA (0.25% trypsin, 1 mM EDTA·4Na) (GibcoBRL) and cells were counted using the hemocytometer with trypan blue (0.4%) exclusion (GibcoBRL). Protein assays were performed to formalize for protein concentration (Bio-Rad, Hercules, CA).

***In vitro* luciferase reporter assay**. The Egr-1 constructs pE425 (596 base pairs containing all CArG elements, no AP-1 sites) and pE660 (the minimal Egr-1 promoter, 115 base pairs no CArG elements) (Datta *et al.,* 1993) were evaluated following sequence confirmation and insertion of the PCR product into the pGL3 basic firefly luciferase reporter plasmid construct (Promega, Madison, WI) by enzyme restriction and ligation. JM109 competent cells (Stratagene, La Jolla, CA) were transformed with these plasmids, endotoxin-free maxipreps (Qiagen, Valencia, CA) were prepared, and product confirmation was performed by PCR, sequencing, enzyme restriction, and gel electrophoresis. Seg-1 and PROb cells were plated at 10⁵ cells/well in 12-well plates and transfected with the firefly luciferase reporter plasmid constructs, pGL3 basic (promoterless, negative control), pGL3 660 (minimal Egr-1 promoter), or pGL3 425 (Egr-1 promoter containing all CArG elements) using the TransFast transfection reagent (Promega). All groups were co-transfected with the Renilla luciferase reporter plasmid construct pRL-TK (HSV thymidine kinase promoter) to normalize for transfection efficiency. 48 hr later, cells were exposed to IR (20Gy) or cisplatin (5 µM). Cells were harvested 6 hr later, and luciferase activity was measured using the Dual-Luciferase reporter assay system (Promega).

***In vivo* measurement of TNF-**α **protein**. Seg-1 or PROb cells (5 x 10⁶/0.1 ml) were injected in the right hind limb of nude mice. Tumor bearing mice were randomized to one of 4 groups: intratumoral (IT) Ad.Null.3511.11D (2 x 10⁸ p.u./10 µl) with intraperitoneal (IP) with normal saline (NS) or cisplatin (8 mg/kg) and IT Ad.Egr.TNF.11D (2 x 10⁸ p.u./10 µl) with IP NS or cisplatin. IP NS or cisplatin treatments were administered after IT vector. Two consecutive IT and IP injections were given. Animals were euthanized, and xenografts were harvested 48 hours following the second IP injection. Xenografts were snap frozen in liquid nitrogen, and homogenized in RIPA buffer (NaCl 150 mM, Tris 10 mM, pH 7.5, EDTA 5 mM, pH 7.5, PMSF 100 mM, Leupeptin 1 µg/ml, Aprotinin 2 µg/ml) using a Brinkman Polytron Homogenizer (Kinematica AG, Lucerne, Switzerland). Following three freeze-thaw lysis cycles, the homogenate was centrifuged at 10,000 rpm (Sorvall RC5C SS34 rotor) for 10 minutes, 4°C. TNF-α levels in the supernatants were measured using ELISA and protein assays were performed (Bio-Rad, Hercules, CA).

***In vivo* regrowth studies**. Seg-1 or PROb cells (5 x 10⁶/0.1 ml) were injected in the right hind limb of nude mice. Tumor bearing mice were assigned to one of 4 groups: intratumoral (IT) Ad.Null.3511.11D (2 x 10⁸ p.u./10 µl) with intraperitoneal (IP) normal saline (NS) or cisplatin (3 mg/kg) and IT Ad.Egr.TNF.11D (2 x 10⁸ p.u./10 µl) with IP NS or cisplatin., IP NS or cisplatin injections were given following the IT vector injection, and 4 consecutive daily IT and IP injections were given. Xenografts were measured every 2 days using calipers and tumor volume was calculated (length x width x thickness)/2. Fractional tumor volumes (V/Vo, Vo = day 0 volume) were calculated and plotted.

**Statistical analysis**. Statistical significance was determined using two-tail student's *t-*test.

### EXAMPLE 2

### In vitro Induction of TNF-α in Human and Rat Tumor Cells Following Infection with Ad.Egr.TNF.11D and Exposure to Cisplatin

Because Egr-1 is induced through the CArG elements of its promoter by ROIs and/or DNA damage, TNF-α production by tumor cells infected with an adenoviral vector in which CArG elements are upstream to a TNF-α cDNA (Ad.Egr.TNF.11D) was analyzed after exposure to cisplatin, a DNA damaging agent that alters cellular redox status (Davis *et al*., 2001). TNF-α production was tested in human esophageal Seg-1 cells and rat colorectal PROb cells following exposure to 5 µM cisplatin. TNF-α concentrations were determined using an ELISA that is specific for human TNF-α. No TNF-α protein was detectable in Seg-1 cell pellets or supernatants from cultures infected with the null vector (Ad.Null.3511.11D), and treated with IR or cisplatin. In contrast, significant levels of TNF-α protein were detected in cultures of Seg-1 cells infected with the Ad.Egr.TNF.11D vector and exposed to IR (5 Gy) at 24, 48 and 72 hrs (768.8 ± 32.6, 593.0 ± 27.6, 746.0 ± 18.5, respectively) compared cells infected with vector alone (269.3 ± 1.9, 167.8 ± 8.4, 260.6 ± 14.9; *P* < 0.001). Combined treatment with Ad.Egr.TNF.11D + IR resulted in a 2.9, 3.5 and 2.9-fold increase in TNF production. A similar induction of TNF-α protein was detected in Seg-1 cells infected with the Ad.Egr.TNF.11D vector and exposed to 5µM cisplatin compared with vector alone at 24 hrs (885.3 ± 28.7), 48 hrs (892.6 ± 21.3) and 72 hrs (901.7 ± 21.7; *P* < 0.001, FIG. 5A). Combined treatment with Ad.Egr.TNF.11D + cisplatin thus resulted in a 3.3, 5.3 and 3.5-fold increase in TNF production.

Comparable experiments were conducted with PROb cell cultures. Again no TNF-α protein was detectable in PROb cell pellets or supernatants from cultures infected with the null vector (Ad.Null.3511.11D) and treated with IR or cisplatin. Significant levels of TNF-α protein were detected in cultures of PROb cells infected with the Ad.Egr.TNF.11D vector and exposed to IR (5 Gy) at 24,48 and 72 hrs (55.1 ± 4.6, 440.5 ± 7.0, 812.7 ± 8.9, respectively) compared cells infected with vector alone (17.9 ± 1.7, 169.7 ± 5.2, 522.5 ± 11.3; *P* < 0.001). Combined treatment with Ad.Egr.TNF.11D + IR resulted in a 3.1, 2.6 and 1.6-fold increase in TNF production: A similar induction of TNF-α protein was detected in Seg-1 cells infected with the Ad.Egr.TNF.11D vector and exposed to 5 µM cisplatin compared with vector alone at 24, 48 and 72 hrs (52.4 ± 0.6, 318.6 ± 30.6, 812.2 ± 11.0; *P* < 0.001, FIG. 5B). Combined treatment with Ad.Egr.TNF.11D + cisplatin resulted in a 2.9, 1.9 and 1.6-fold increase in TNF production. These findings from the Seg-1 and PROb cell lines demonstrate that IR and cisplatin induce TNF-α expression by activating the Egr-1 promoter.

With a selective tumor-targeting vector, a cisplatin inducible genetic construct enhances the effects of cisplatin, in this case by TNF-α. Cisplatin and TNF-α have different mechanisms of cell killing and therefore, cells resistant to cisplatin may be sensitive to TNF-α and vice versa. Also, necrosis is induced by high intratumoral concentrations of TNF-α by damage to the tumor microvasculature, which may be useful in treatment of TNF-α and cisplatin resistant tumors. the cisplatin/Ad.Egr.TNF .11D strategy thus is an effective therapy for localized tumors not effectively treated with radiotherapy or surgery. Also, Ad.Egr.TNF.11D may enhance the local effects of combination chemo-radiation therapy.

### EXAMPLE 3

### CArG Elements of the Egr-1 Promoter Mediate Induction of TNF-α by Cisplatin

To study whether the CArG elements of the Egr-1 promoter are inducible by cisplatin, Egr-1 promoter activity was assessed by measuring activation of the luciferase reporter gene in Seg-1 and PROb cells co-transfected with the firefly luciferase reporter plasmid constructs pGL3 basic (negative control), pGL3 660 (consisting only of the minimal Egr-1 promoter, no CArG elements), or pGL3 425 (consisting of all the CArG elements, no AP-1 sites), and the Renilla luciferase reporter plasmid construct pRL-TK. Minimal luciferase activity (LA) was detectable in Seg-1 cells transfected with the pGL3 basic plasmid construct (LA = 0.01- 0.02) or with the pGL3 660 plasmid construct (LA = 0.10-0.18). However, Seg-1 cells transfected with the pGL3 425 plasmid construct exhibited a 2.4-fold increase (*P*=0.005) in relative luciferase activity (LA =15.07) following exposure to IR (20 Gy) compared to untreated control (LA = 6.37) and a 2.0-fold increase (*P*=0.005) in luciferase activity (LA = 2.89) following exposure to cisplatin (50 µM) compared with untreated control (FIG. 6A).

Similar results were obtained with the PROb cell line. Minimal luciferase activity was detectable in PROb cells transfected with the pGL3 basic plasmid construct (LA = 0.21 - 0.30) or with the pGL3 660 plasmid construct (LA = 0.76 - 1.84). PROb cells transfected with the pGL3 425 plasmid construct exhibited a 4.2-fold increase (*P*=0.004) in luciferase activity (LA = 57.75) following exposure to IR (20 Gy) compared to untreated control (LA = 13.69) and a 3.6-fold increase (*P*=0.01) in luciferase activity (LA = 49.40) following exposure to cisplatin (50 µM) compared with untreated control (FIG. 6B). These data demonstrate that CArG elements of the Egr-1 promoter are inducible by cisplatin and mediate the transcriptional activation of the chimeric Egr-1.TNF-α gene.

### EXAMPLE 4

### Induction of TNF-α in Human and Rat Tumor Xenografts Following Treatment with Ad.Egr.TNF.11D and Cisplatin

TNF-α induction by cisplatin was analyzed following infection of human and rodent tumors with the Ad.Egr.TNF.11D vector. Xenografts of Seg-1 or PROb cells growing the hind limbs of athymic nude mice were injected intratumorally (IT) with Ad.Nu11.3511.11D or Ad.Egr.TNF.11D. Tumor bearing mice were injected IP with either normal saline (NS) or cisplatin (3 mg/kg). TNF-α concentration in tumor homogenates was quantified using ELISA.

No TNF-α protein was detected in Seg-1 tumor homogenates following injection of the Ad.Null.3511.11D vector and systemic treatment with either NS or cisplatin. A significant increase (3.5-fold) in intratumoral TNF-α protein was observed following combined treatment with Ad.Egr.TNF.11D + cisplatin (1294.0 ± 438.5 pg/mg) compared with treatment with vector alone (366.5 ± 52.6 pg/mg; *P*<0.05, FIG. 7A).

No TNF-α protein was detected in PROb tumor homogenates following injection: of Ad.Null.3511.11D vector and systemic treatment with either NS or cisplatin. However, a significant increase (2.7-fold) in intratumoral TNF-α protein was observed following combined treatment with Ad.Egr.TNF:11D + cisplatin (878.6 ± 61.9 pg/mg) compared to treatment with vector alone (321.4 ± 27.7 pg/mg; *P*<0.001, FIG. 7B). These findings demonstrate *in vivo* induction of TNF-α protein by cisplatin and verify that the TNF-α protein is a product of the Ad.Bgr.TNF.11D vector rather than the tumor tissue.

### EXAMPLE 5

### Cisplatin Inducible Ad.Egr.TNF.11D Enhances Treatment of Human and Rat Xenografts

Potential antitumor effects of chemo-inducible Ad.Egr.TNF.11D and cisplatin were examined in Seg-1 and PROb xenografts. In the Seg-1 studies, mean tumor volume on day 0 (initiation of treatment) was 381.3 ± 10.8 mm³ (n = 48, 12 mice per treatment group). Xenografts were injected IT with either Ad.Null.3511.11D or Ad.Egr.TNF.11D. Mice were injected IP with either NS or cisplatin. Control tumors (Ad.Nu11.3511.11D + NS) doubled in size by day 4 and exhibited a 4.7 fold increase in mean tumor volume by day 14. A similar growth pattern was observed in tumors treated with the Ad.Egr.TNF.11D vector + NS (2.0-fold increase at day 4 and 3.8-fold increase in mean volume at day 14). Significant tumor regression was observed in the tumors receiving combined treatment with Ad.Egr.TNF.11D + cisplatin compared with tumors treated with the null vector + cisplatin on days 4 (*P*=0.045), 6 (*P*<0.005), 8 (*P*<0.002), 10 (*P*<0.001), 12 (*P*<0.004), and 14 (*P*<0.021), (FIG. 8A).

In the PROb studies, mean tumor volume on day 0 was 244.2 ± 6.2 mm³ (n = 40, 10 mice per treatment group). Control tumors (Ad.Null.3511.11D + NS) grew steadily doubling in size by day 4, exhibiting a 4.4 fold increase in mean tumor volume by day 14. A similar growth pattern was observed for tumors treated with the Ad.Egr.TNF.11D vector + NS (1.6-fold increase at day 4 and 3.6-fold increase in mean volume at day 14). Significant tumor regression was observed in the tumors receiving combined treatment with Ad.Egr.TNF.11D + cisplatin compared with tumors treated with the null vector + cisplatin on days 4 (*P*=0.045), 6 (*P*<0.001), 8 (*P*=0.048), 10 (*P*<0.001), 12 (*P*<0.001), and 14 (*P*=0.002), (FIG. 8B). Taken together, these data support an antitumor interaction between cisplatin and Ad.Egr.TNF.11D in xenografts of human and rodent origin. These findings are consistent with, and supported by, TNF-α induction by cisplatin observed in the *in vitro* and *in vivo* experiments. Although toxicity was observed following treatment with cisplatin, no additional toxicity was observed following combined treatment with cisplatin and Ad.Egr.TNF.11D.

Thus, cisplatin, a commonly employed chemotherapeutic agent which stimulates ROI production, induces the production of TNF-α in human and rodent cancer cells infected with an adenoviral vector encoding the CArG elements of the Egr-1 promoter ligated upstream to a cDNA encoding TNF-α. Significant antitumor effects of both TNF-α and cisplatin were observed in both experimental tumor systems. Thus, the present invention provides a new approach that combines the use of chemotherapeutic agents, such as cisplatin, with the temporal and spatial control of gene therapy using antitumor genes.

For most common human neoplasms, grossly visible tumors are not effectively treated with most standard chemotherapeutic agents. The transcriptional targeting strategy such as the Egr1-TNF-α and cisplatin is useful when it is possible to infuse or directly inject gross tumors, even in the presence of micrometastases, since the vector/cisplatin combination is effective against gross tumor and cisplatin against micrometastatic disease. The direct injection of tumors should be improved with the recent advances in radiographic imaging analysis of tumor, e.g. PET scans, combined with CT image reconstruction. Additionally, recent developments in the targeting of viral vectors to tumors may provide additional specificity to chemoinducible gene therapy of metastatic cancer.

### J. References

U.S. Patent 4,664,911
U.S. Patent 4,684,611
U. S. Patent 4,792,447
U.S. Patent 4,797,368
U.S. Patent 4,952,500
U.S. Patent 5,045,451
U.S. Patent 5,139,941
U.S. Patent 5,302,523
U. S. Patent 5,322,783
U. S. Patent 5,354,855
U. S. Patent 5,359,046
U. S. Patent 5,384,253
U. S. Patent 5,464,765
U. S. Patent 5,538,877
U.S. Patent 5,538,880
U. S. Patent 5,550,318
U. S. Patent 5,563,055
U. S. Patent 5,578,706
U. S. Patent 5,580,859
U.S. Patent 5,589,466
U. S. Patent 5,591,616
U.S. Patent 5,610,042
U. S. Patent 5,656,610
U.S. Patent 5,686,072
U.S. Patent 5,702,932
U. S. Patent 5,736,524
U. S. Patent 5,767,072
U.S. Patent 5,780,448
U. S. Patent 5,925,565
U.S. Patent 5,935,819
U. S. Patent 5,945,100,
U.S. Patent 5,981,274
U.S. Patent 5,994,136
U. S. Patent 5,994,624
U. S. Patent 6,013,516

Arap et al., Cancer Res., 55:1351-1354, 1995.
Ausubel et al., In: Current Protocols in Molecular Biology, John, Wiley and Sons, Inc., 1994. Baichwal and Sugden, In: Gene Transfer, Kucherlapati R, ed., New York, Plenum Press, pp. 117-148, 1986.
Bajorin et al., Proc. Annu. Meet. Am. Soc. Clin. Oncol., 7:A967, 1988.
Bedzyk et al., J. Biol. Chem., 265:18615,1990
Blomer et al., J Virol. 71(9): 6641-6649, 1997
Bonavida et al., Gynecol Oncol, 38:333-9, 1990.
Burbage et al., Leuk Res, 21(7):681-690, 1997.
Bussemakers et al., Cancer Res., 52:2916-2922, 1992.
Caldas et al., Nat. Genet., 8:27-32, 1994.
Carbonelli et al. FEMS Microbiol Lett. 177(1):75-82, 1999.
Casey et al., Oncogene, 6:1791-1797, 1991.
Chandler et al., Proc Natl Acad Sci USA, 94(8):3596-3601, 1997.
Chaudhary et al. Proc. Natl. Acad. Sci., 87:9491,1990
Chen and Okayama, Mol. Cell Biol., 7:2745-2752, 1987.
Cheng et al., Cancer Res., 54:5547-5551, 1994.
Cheung et al., Arch Biochem Biophys, 305(2):563-569,1993.
Cocea, Biotechniques, 23:814-816, 1997.
Cotten et al., Proc Natl Acad Sci USA, 89(13):6094-6098, 1992.
Coupar et al., Gene, 68:1-10, 1988.
Curiel, In: Viruses in Human Gene Therapy, J.-M.H. Vos (Ed.), Carolina Academic Press, Durham, NC, pp 179-212, 1994.
Datta et al., Proc Natl Acad Sci USA, 90:2419-22, 1993.
Davis et al., JPharmacol Exp Ther, 296:1-6, 2001.
Demetri et al., J Clin Oncol, 7:1545-53, 1989.
Duan et al., JNeurooncol, 52:23-36, 2001.
Edelman and Crossin, Annu. Rev. Biochem., 60:155-190, 1991.
Edelman, Annu. Rev. Biochem., 54:135-169, 1985.
Fechheimer et al., Proc. Natl. Acad. Sci. USA, 84:8463-8467, 1987.
Fiers, FEBS Letters, 285(2):199-212, 1991.
Forster and Symons, Cell, 49:211-220, 1987.
Fraley et al., Proc. Natl. Acad. Sci. USA, 76:3348-3352, 1979.
Friedmann, Science, 244:1275-1281,1989.
Frixen et al., J. Cell Biol., 113:173-185, 1991.
Gerlach et al., Nature (London), 328:802-805, 1987
Ghosh and Bachhawat, In: Liver diseases, targeted diagnosis and therapy using specific receptors and ligands, (Wu G, Wu C ed.), New York: Marcel Dekker, pp. 87-104,1991.
Giancotti and Ruoslahti, Cell, 60:849-859, 1990.
Gonzalez-Zulueta et al., Cancer Research, 55(20):4531-4535, 1995.
Goodbourn and Maniatis, Proc. Natl. Acad. Sci. USA, 85:1447, 1988.
Gopal, Mol. Cell Biol., 5:1188-1190,1985.
Gorczyca et al., Cancer Res., 53:1945-1951, 1993.
Graham and Van Der Eb, Virology, 52:456-467, 1973.
Grunhaus and Horwitz, Seminar in Virology, 3:237-252, 1992.
Hall, Radiobiology for the Radiologist, Harper and Row, 1988.
Hall, Radiobiology for the Radiologist, Harper and Row, 1994.
Harland and Weintraub, J. Cell Biol., 101:1094-1099,1985.
Havell et al., J Exp Med, 167:1067-85, 1988.
Herman et al., Cancer Research, 55(20):4525-4530, 1995.
Hollstein et al., Science, 253:49-53, 1991.
Horwich et al. J. Virol., 64:642-650, 1990.
Hussussian et al., Nature Genetics, 15-21, 1994.
Inouye et al., Nucl. Acids Res., 13:3101-3109, 1985.
Irie and Morton, Proc. Nat'l Acad. Sci. USA 83:8694-8698, 1986
Johnson and Stevenson, In: Cancer.Principles and Practice of Oncology (ed. DeVita, V.T., Hellman and Rosenberg,) 376-88, 2001.
Joyce, Nature, 338:217-244, 1989.
Kamb et al., Nature Genetics, 8:22-26, 1994.
Kamb et al., Science, 2674:436-440, 1994.
Kaneda et al., Science, 243:375-378, 1989.
Kartalou and Essigmann, Mutat Res, 478:23-43, 2001.
Kato et al, J. Biol. Chem., 266:3361-3364, 1991.
Kelleher and Vos, Biotechniques, 17(6):1110-1117, 1994.
Kim and Cook, Proc. Natl. Acad. Sci. USA, 84:8788-8792, 1987.
Kucuk et al., Am J Clin Oncol, 23:371-5, 2000.
Levenson et al., Human Gene Therapy, 9:1233-1236, 1998.
Lebkowski et al., Mol Cell Biol, 8(10):3988-3996, 1988.
Lidor et al., Am J Obstet Gynecol;177(3):579-585, 1997.
Lin and Guidotti, J. Biol. Chem., 264:14408-14414, 1989:
Laughlin et al., J. Virol., 60(2):515-524, 1986.
McLaughlin et al., J. Virol., 62(6):1963-1973, 1988.
Macejak and Sarnow, Nature, 353:90-94, 1991.
Mann et al., Cell, 33:153-159, 1983.
Massuda et al., Proc Natl Acad Sci USA, 94(26):14701-14706, 1997.
Matsura et al., Brit. J. Cancer, 66:1122-1130, 1992.
Mauceri et al., Int J Cancer, 97:410-5, 2002.
Merlo et al., Nat Med., (7):633-4, 1995.
Michel and Westhof, J. Mol. Biol., 216:585-610, 1990.
Miksicek et al., Cell, 46:203, 1986.
Mizukami et al., Virology, 217:124-130, 1996.
Mori et al., Cancer Res., 54:3396-3397, 1994.
Morton and Ravindranath, In Tumor Immunology, Dalgleish (ed.), London: Cambridge University Press, 1-55, 1996.
Morton et al., Ann. Surg., 216:463-482, 1992.
Muzyczka, Curr. Top. Microbiol. Immunol., 158:97-129,1992.
Myers, EPO 0273085.
Nabel et al., Science, 244:1342-1344, 1989.
Nakamoto et al., Anticancer Res, 20:4087-96, 2000.
Naldini et al., Science, 272(5259):195, 1996
Nicolas and Rubenstein, In: Vectors: A survey of molecular cloning vectors and their uses, Rodriguez and Denhardt (eds.), Stoneham: Butterworth, pp. 493-513, 1988.
Nicolau and Sene, Biochim. Biophys. Acta, 721:185-190, 1982.
Nicolau et al., Methods Enzymol., 149:157-176, 1987.
Nobri et al., Nature, 368:753-756, 1995.
Obrador et al., Curr Pharm Biotechnol, 2:119-30, 2001.
Obrink, BioEssays, 13:227-233, 1991.
Odin and Obrink, Exp. Cell Res., 171:1-15, 1987.
Okamoto et al., Proc. Nat'l Acad. Sci. USA, 91:11045-11049, 1994.
Old, Science, 230:630-2, 1985.
Omirulleh et al., Plant Mol. Biol., 21:415-28, 1993.
Orlow et al., Cancer Res., 54:2848-2851,1994.
Paskind et al., Virology, 67:242-248, 1975.
PCT 94/09699
PCT 95/06128
Pelletier and Sonenberg, Nature, 334:320-325, 1988.
Perales et al., Proc. Natl. Acad. Sci. 91:4086-4090,1994.
Potrykus et al., Mol. Gen. Genet., 199:183-188, 1985.
Potter et al., Proc. Nat'l Acad. Sci. USA, 81:7161-7165, 1984.
Ravindranath and Morton, Intern. Rev. Immunol., 7: 303-329, 1991.
Reinhold-Hurek and Shub, Nature, 357:173-176, 1992.
Remington's Pharmaceutical Sciences, 15th ed., pp. 1035-1038 and 1570-1580.
Ridgeway, In: Vectors: A survey of molecular cloning vectors and their uses, Rodriguez RL, Denhardt DT, ed., Stoneham:Butterworth, pp. 467-492,1988.
Rippe et al., Mol. Cell Biol., 10:689-695, 1990.
Robaye et al., Am J Pathol, 138:447-53, 1991.
Rosenberg, P., Autotransfusion (editorial)"Duodecim., 106 (14) p1027-9, 1990.
Roux et al., Proc. Natl Acad. Sci. USA, 86:9079-9083, 1989.
Sarver et al., Science, 247:1222-1225, 1990.
Scanlon et al., Proc Natl Acad Sci USA, 88:10591-10595, 1991.
Serrano et al., Nature, 366:704-707, 1993.
Serrano et al., Science, 267:249-252, 1995.
Slungaard et al., J Exp Med, 171:2025-41, 1990.
Smith and Rutledge, Natl Cancer Inst Monogr, 42:169-172, 1975.
Spriggs et al., JNatl Cancer Inst, 80:1039-44, 1988.
Staba et al., Gene Therapy, 5:293-300, 1998.
Takahashi et al., Cancer Res., 52:734-736, 1992.
Tartaglia et al, Cell, 74:845-53, 1993.
Temin, In: Gene Transfer, Kucherlapati (ed.), New York: Plenum Press, pp. 149-188, 1986.
Thom et al., J Clin Oncol, 13:264-73, 1995.
Tratschin et al., Mol. Cell. Biol., 4:2072-2081,1984.
Tur-Kaspa et al., Mol. Cell Biol., 6:716-718, 1986.
Umbas et al., Cancer Res., 52:5104-5109, 1992.
Wagner et al., Proc. Natl. Acad. Sci. 87(9):3410-3414, 1990.
Watanabe et al., Cancer Res, 48:2179-83, 1988.
Weichselbaum et al., Acta Oncol 40, 735-8, 2001.
Weichselbaum et al., Int JRadiat Oncol Biol Phys, 30:229-34, 1994.
Weinberg, Science, 254:1138-1145, 1991.
Wilson et al., Science, 244:1344-1346, 1989.
Wong et al., Gene, 10:87-94, 1980.
Wu and Wu, Adv. Drug Delivery Rev., 12:159-167, 1993.
Wu and Wu, Biochem., 27:887-892, 1988.
Wu and Wu, J. Biol. Chem., 262:4429-4432,1987.
Young et al., NEngl J Med. 7:299(23):1261-1266, 1978.
Zufferey et al., Biotechnol, 15(9):871-875, 1997

## Claims

1. An expression vector in combination with at least one free radical-inducing DNA damaging compound for use in therapy, wherein said expression vector comprises a nucleic acid segment encoding a protein of interest, said nucleic acid segment being positioned under the control of an Egr-1 promoter, whereby said DNA damaging compound induces expression of said protein of interest from said Egr-1 promoter in a cell,
wherein said free radical-inducing DNA damaging compound is selected from the group consisting of a platinum compound, cisplatin, nitrogen mustard, cytoxan, cyclophosphamide, mitomycin c, iphosphamide, bleomycin, actinomycin,carboplatinum, busulfan, bcnu, ccnu, hexamethylmelamineoxaliplatin, mitoxantrone, 5-fluorouracil, gemcitabine, and paclitaxel.

2. The combination of claim 1, wherein said expression vector is further in combination with at least a second free-radical inducing DNA damaging compound.

3. The combination of claim 1, wherein said expression vector is further in combination with a cancer chemotherapeutic compound.

4. The combination of claim 1, wherein said cell is a cancer cell.

5. The combination of claim 4, wherein said cancer cell is a lung cancer cell, prostate cancer cell, ovarian cancer cell, testicular cancer cell, brain cancer cell, skin cancer cell, colon cancer cell, gastric cancer cell, esophageal cancer cell, tracheal cancer cell, head & neck cancer cell, pancreatic cancer cell, liver cancer cell, breast cancer cell, ovarian cancer cell, lymphoid cancer cell, leukemia cell, cervical cancer cell, or vulvar cancer cell.

6. The combination of claim 1, wherein said expression vector further comprises a polyadenylation signal operably linked to said nucleic acid segment.

7. The combination of claim 1, wherein said expression vector is a viral vector.

8. The combination of claim 7, wherein said viral vector is an adenoviral vector, an adeno-associated viral vector, a retroviral vector, a vaccinia viral vector, or a herpesviral vector.

9. The combination of claim 7, wherein said viral vector is lacking one or more viral genes, thus rendering said viral vector non-replicative.

10. The combination of claim 1, wherein said protein of interest is a tumor suppressor, an inducer of apoptosis, an enzyme, a cytokine, or a toxin.

11. The combination of claim 10, wherein said enzyme is thymidine kinase, cytosine deaminase, hypoxanthine guanine phosphoribosyl transferase.

12. The combination of claim 10, wherein said cytokine is TNF-α.

13. The combination of claim 1 for treatment of a human.

14. The use of an expression vector comprising a nucleic acid segment encoding a cancer therapeutic protein, said nucleic acid segment being positioned under the control of an Egr-1 promoter, in the manufacture of a medicament for the treatment of a hyperproliferative disease in a subject; wherein said treatment comprises administering said expression vector to said subject in combination with a free radical-inducing DNA damaging compound, whereby said DNA damaging compound induces expression of said cancer therapeutic protein from said Egr-1 promoter, thereby treating said hyperproliferative disease in said subject,
and wherein the free radical-inducing DNA damaging compound is selected from the group consisting of a platinum compound, cisplatin, nitrogen mustard, cytoxan, cyclophosphamide, mitomycin c, iphosphamide, bleomycin, actinomycin,carboplatinum, busulfan, bcnu, ccnu, hexamethylmelamineoxaliplatin, mitoxantrone, 5-fluorouracil, gemcitabine, and paclitaxel.

15. The use according to claim 14, wherein said hyperproliferative disease is cancer.

16. The use of claim 14, wherein said expression vector is suitable for local or regional delivery to a tumor located in said subject.

17. The use of claim 14, wherein said expression vector is suitable for systemic delivery.

18. The use of claim 14, wherein said expression vector is suitable for delivery via intratumoral injection or by direct injection into tumor vasculature.

19. The use of claim 14, wherein said expression vector is for administration after said DNA damaging compound or prior to said DNA damaging compound.

20. The use of claim 14, wherein said expression vector and said DNA damaging compound are for simultaneous administration.

21. The use of claim 14, wherein said treatment comprises administering said expression vector at least twice.

22. The use of claim 14, wherein said treatment comprises administering said DNA damaging compound at least twice.

23. The use of claim 14 wherein said medicament is for inhibiting tumor cell growth, for killing a tumor cell, for inhibiting tumor cell metastasis, for reducing tumor burden, or for rendering an inoperable tumor operable in said subject.

24. The use of claim 14, wherein the cancer therapeutic protein is TNF-α.

## Patentansprüche

1. Expressionsvektor in Kombination mit zumindest einer, freies Radikal induzierenden, DNA zerstörenden Verbindung zur Verwendung in Therapie, worin der Expressionsvektor einen Nucleinsäureabschnitt umfasst, der für ein Protein von Interesse kodiert, wobei der Nucleinsäureabschnitt unter die Kontrolle eines Egr-1-Promotors gestellt ist, wodurch die DNA zerstörende Verbindung die Expression des Proteins von Interesse aus dem Egr-1-Promotor in einer Zelle induziert,
worin die freies Radikal induzierende, DNA zerstörende Verbindung aus der aus einer Platinverbindung, Cisplatin, Stickstofflost, Cytoxan, Cyclophosphamid, Mitomycin c, Iphosphamid, Bleomycin, Actinomycin, Carboplatin, Busulfan, bcnu, ccnu, Hexamethylmelaminoxaliplatin, Mitoxantron, 5-Fluoruracil, Gemcitabin und Paclitaxel bestehenden Gruppe ausgewählt ist.

2. Kombination nach Anspruch 1, worin der Expressionsvektor weiters in Kombination mit zumindest einer zweiten, freies Radikal induzierenden, DNA zerstörenden Verbindung vorliegt.

3. Kombination nach Anspruch 1, worin der Expressionsvektor weiters in Kombination mit einer krebschemotherapeutischen Verbindung vorliegt.

4. Kombination nach Anspruch 1, worin die Zelle eine Krebszelle ist.

5. Kombination nach Anspruch 4, worin es sich bei der Krebszelle um eine Lungenkrebszelle, eine Prostatakrebszelle, eine Eierstockkrebszelle, eine Hodenkrebszelle, eine Hirnkrebszelle, eine Hautkrebszelle, eine Dickdarmkrebszelle, eine Magenkrebszelle, eine Ösophagenkrebszelle, eine Luftröhrenkrebszelle, eine Kopf-Hals-Krebszelle, eine Bauchspeicheldrüsenkrebszelle, eine Leberkrebszelle, eine Brustkrebszelle, eine Eierstockkrebszelle, eine Lymphkrebszelle, eine Leukämiezelle, eine Gebärmutterhalskrebszelle oder eine Vulvakrebszelle handelt.

6. Kombination nach Anspruch 1, worin der Expressionsvektor weiters ein Polyadenylierungssignal umfasst, das operabel mit dem Nucleinsäureabschnitt verbunden ist.

7. Kombination nach Anspruch 1, worin es sich bei dem Expressionsvektor um einen Virusvektor handelt.

8. Kombination nach Anspruch 7, worin es sich bei dem Virusvektor um einen Adenovirusvektor, einen adenoassoziierten Virusvektor, einen Retrovirusvektor, einen Vakziniavirusvektor oder einen Herpesvirusvektor handelt.

9. Kombination nach Anspruch 7, worin dem Virusvektor ein oder mehrere Virengene fehlen, wodurch der Virusvektor nicht replizierbar ist.

10. Kombination nach Anspruch 1, worin es sich bei dem Protein von Interesse um einen Tumorsuppressor, einen Induktor von Apoptose, ein Enzym, ein Zytokin oder ein Toxin handelt.

11. Kombination nach Anspruch 10, worin es sich bei dem Enzym um Thymidinkinase, Cytosindesaminase oder Hypoxanthinguaninphosphoryltransferase handelt.

12. Kombination nach Anspruch 10, worin es sich bei dem Zytokin um TNFα handelt.

13. Kombination nach Anspruch 1 zur Behandlung eines Menschen.

14. Verwendung eines Expressionsvektors, der einen Nucleinsäureabschnitt umfasst, der für ein krebstherapeutisches Protein kodiert, wobei der Nucleinsäureabschnitt unter die Kontrolle eines Egr-1-Promotors gestellt ist, zur Herstellung eines Medikaments zur Behandlung einer hyperproliferativen Erkrankung bei einem Individuum, worin die Behandlung das Verabreichen des Expressionsvektors an das Individuum in Kombination mit einer freies Radikal induzierenden, DNA zerstörenden Verbindung umfasst, wodurch die DNA zerstörende Verbindung die Expression des krebstherapeutischen Proteins aus dem Egr-1-Promotor induziert und damit die hyperproliferative Erkrankung bei dem Individuum behandelt wird, und worin die freies Radikal induzierende, DNA zerstörende Verbindung aus der aus einer Platinverbindung, Cisplatin, Stickstofflost, Cytoxan, Cyclophosphamid, Mitomycin c, Iphosphamid, Bleomycin, Actinomycin, Carboplatin, Busulfan, bcnu, ccnu, Hexamethylmelaminoxaliplatin, Mitoxantron, 5-Fluoruracil, Gemcitabin und Paclitaxel bestehenden Gruppe ausgewählt ist.

15. Verwendung nach Anspruch 14, worin es sich bei der hyperproliferativen Erkrankung um Krebs handelt.

16. Verwendung nach Anspruch 14, worin der Expressionsvektor zur lokalen oder regionalen Zufuhr zu einem in dem Individuum befindlichen Tumor geeignet ist.

17. Verwendung nach Anspruch 14, worin der Expressionsvektor zur systemischen Zufuhr geeignet ist.

18. Verwendung nach Anspruch 14, worin der Expressionsvektor zur Zufuhr durch intratumorale Injektion oder durch direkte Injektion in die Tumorgefäßstruktur geeignet ist.

19. Verwendung nach Anspruch 14, worin der Expressionsvektor zur Verabreichung nach der DNA zerstörenden Verbindung oder vor der DNA zerstörenden Verbindung gedacht ist.

20. Verwendung nach Anspruch 14, worin der Expressionsvektor und die DNA zerstörende Verbindung zur gleichzeitigen Verabreichung gedacht sind.

21. Verwendung nach Anspruch 14, worin die Behandlung das zumindest zweimalige Verabreichen des Expressionsvektors umfasst.

22. Verwendung nach Anspruch 14, worin die Behandlung das zumindest zweimalige Verabreichen der DNA zerstörenden Verbindung umfasst.

23. Verwendung nach Anspruch 14, worin das Medikament bei dem Individuum zur Hemmung der Tumorzellenvermehrung, zur Tötung einer Tumorzelle, zur Hemmung von Tumorzellenmetastasen, zur Reduktion der Tumorlast oder zur Überführung eines nicht operablen Tumors in einen operablen dient.

24. Verwendung nach Anspruch 14, worin es sich bei dem krebstherapeutischen Protein um TNFα handelt.

## Revendications

1. Vecteur d'expression en combinaison avec au moins un composé endommageant l'ADN induisant des radicaux libres pour utilisation en thérapie, où ledit vecteur d'expression comprend un segment d'acide nucléique codant pour une protéine d'intérêt, ledit segment d'acide nucléique étant positionné sous la commande d'un promoteur Egr-1, moyennant quoi ledit composé endommageant l'ADN induit l'expression de ladite protéine d'intérêt dudit promoteur Egr-1 dans une cellule,
où ledit composé endommageant l'ADN induisant des radicaux libres est sélectionné dans le groupe consistant en un composé de platine, cisplatine, moutarde azotée, cytoxane, cyclophosphamide, mitomycine c, iphosphamide, bléomycine, actinomycine, carboplatine, busulfane, bcnu, ccnu, hexaméthylmétamineoxaliplatine, mitoxantrone, 5-fluorouracil, gemcitabine et pactitaxel.

2. Combinaison selon la revendication 1, où ledit vecteur d'expression est en outre en combinaison avec au moins un deuxième composé endommageant l'ADN induisant des radicaux libres.

3. Combinaison selon la revendication 1, où ledit vecteur d'expression est en outre en combinaison avec un composé chimiothérapique de cancer.

4. Combinaison selon la revendication 1, où ladite cellule est une cellule cancéreuse.

5. Combinaison selon la revendication 4, où ladite cellule cancéreuse est une cellule du cancer du poumon, une cellule du cancer de la prostate, une cellule du cancer des ovaires, une cellule du cancer des testicules, une cellule du cancer du cerveau, une cellule du cancer de la peau, une cellule du cancer du côlon, une cellule du cancer gastrique, une cellule du cancer de l'oesophage, une cellule du cancer de la trachée, une cellule du cancer de la tête et du cou, une cellule du cancer du pancréas, une cellule du cancer du foie, une cellule du cancer du sein, une cellule du cancer des ovaires, une cellule du cancer lymphoïde, une cellule de leucémie, une cellule du cancer cervical ou une cellule du cancer de la vulve.

6. Combinaison selon la revendication 1, où ledit vecteur d'expression comprend en outre un signal de polyadénylation fonctionnellement lié audit segment d'acide nucléique.

7. Combinaison selon la revendication 1, où ledit vecteur d'expression est un vecteur viral.

8. Combinaison selon la revendication 7, où ledit vecteur viral est un vecteur adénoviral, un vecteur viral adéno-associé, un vecteur rétroviral, un vecteur viral de la vaccine ou un vecteur viral de l'herpès.

9. Combinaison selon la revendication 7, où il manque audit vecteur viral un ou plusieurs gènes viraux en rendant ainsi ledit vecteur viral non-réplicatif.

10. Combinaison selon la revendication 1, où ladite protéine d'intérêt est un suppresseur de tumeur, un inducteur d'apoptose, une enzyme, une cytokine ou une toxine.

11. Combinaison selon la revendication 10, où ladite enzyme est la thymidine kinase, cytosine désaminase, hypoxanthine guanine phosphoribosyl transférase.

12. Combinaison selon la revendication 10, où ladite cytokine est TNF-α.

13. Combinaison selon la revendication 1 pour le traitement d'un être humain.

14. Utilisation d'un vecteur d'expression comprenant un segment d'acide nucléique codant pour une protéine thérapeutique du cancer, ledit segment d'acide nucléique étant positionné sous le contrôle d'un promoteur Egr-1, dans la fabrication d'un médicament pour le traitement d'une maladie hyperproliférative chez un sujet; où ledit traitement comprend l'administration dudit vecteur d'expression audit sujet en combinaison avec un composé endommageant l'ADN induisant des radicaux libres, moyennant quoi ledit composé endommageant l'ADN induit l'expression de ladite protéine thérapeutique du cancer dudit promoteur Egr-1, en traitant ainsi ladite maladie hyperproliférative chez ledit sujet, et où le composé endommageant l'ADN induisant des radicaux libres est sélectionné dans le groupe consistant en un composé de platine, cisplatine, moutarde azotée, cytoxane, cyclophosphamide, mitomycine, iphosphamide, bléomycine actinomycine, carboplatine, busulfane, bcnu, ccnu, hexaméthylmélamineoxaliplatine, mitoxantrone, 5-fluorouracil, gemcitabine et paclitaxel.

15. Utilisation selon la revendication 14, où ladite maladie hyperproliférative est le cancer.

16. Utilisation selon la revendication 14, où ledit vecteur d'expression convient pour une délivrance locale ou régionale à une tumeur située dans ledit sujet.

17. Utilisation selon la revendication 14, où ledit vecteur d'expression convient pour une délivrance systémique.

18. Utilisation selon la revendication 14, où ledit vecteur d'expression convient pour la délivrance par injection intratumorale ou par injection directe dans la vasculature de la tumeur.

19. Utilisation selon la revendication 14, où ledit vecteur d'expression est pour l'administration après ledit composé endommageant l'ADN ou avant ledit composé endommageant l'ADN.

20. Utilisation selon la revendication 14, où ledit vecteur d'expression et ledit composé endommageant l'ADN sont prévus pour une administration simultanée.

21. Utilisation selon la revendication 14, où ledit traitement comprend l'administration dudit vecteur d'expression au moins deux fois.

22. Utilisation selon la revendication 14, où ledit traitement comprend l'administration dudit composé endommageant l'ADN au moins deux fois.

23. Utilisation selon la revendication 14, où ledit médicament est pour l'inhibition de la croissance des cellules tumorales, pour tuer une cellule de tumeur, pour inhiber une métastase des cellules de la tumeur, pour réduire la charge de la tumeur et pour rendre une tumeur inopérable opérable chez ledit sujet.

24. Utilisation selon la revendication 14, où la protéine thérapeutique de cancer est le TNF-α.
